# FASCICULE DE BREVET EUROPEEN

(11) **EP 0 861 830 B1**
(45) Date de publication et mention de la délivrance du brevet: **18.12.2002**
(21) Numéro de dépôt: 98400298.0
(22) Date de dépôt: 10.02.1998
(51) Int. Cl.: C07D 207/32, C07D 207/34, C07D 207/48, A61K 7/13

(54) **Utilisation de dérivés pyrroliques de la 1,4-naphtoquinone et du 1,4-dihydroxynaphtalene en teinture des matières kératiniques, nouveaux composés et compositions les contenant**
Verwendung von 1,4-Naphthochinon- und 1,4-dihydroxynaphthalin-Pyrrolderivaten zum Färben von Keratinsubstanzen, Verbindungen und diese enthaltende Zusammensetzungen
Use of 1,4-naphthoquinone and 1,4-dihydroxynaphthalene pyrrole derivatives for dyeing keratinous material, compounds and compositions containing them

(30) Priorité: 27.02.1997 FR 9702370
(43) Date de publication de la demande: 02.09.1998
(73) Titulaire: L'OREAL, 75008 Paris (FR)
(72) Inventeur: Genard, Sylvie, 75012 Paris (FR); Andrean, Hervé, 75014 Paris (FR); Hocquaux, Michel, 75012 Paris (FR)
(74) Mandataire: Miszputen, Laurent

(56) Documents cités:
- FR-A- 2 537 433
- PATENT ABSTRACTS OF JAPAN vol. 097, no. 005, 30 mai 1997 & JP 09 006028 A (MITA IND CO LTD), 10 janvier 1997,
- YOSHIDA, SEIGO ET AL: "Synthesis of 2,3-diarylquinone by palladium catalyzed cross-coupling of dibromoquinones with heteroarylstannanes" CHEM. LETT. (1996), (2), 139-40 CODEN: CMLTAG;ISSN: 0366-7022, 1996, XP002044650
- MATSUOKA, MASARU ET AL: "Syntheses of 3,4-bisaryl-3-cyclobutene-1,2-diones and related heterocycles" DYES PIGM. (1991), 16(4), 309-15 CODEN: DYPIDX;ISSN: 0143-7208, 1991, XP002044651
- YOSHIDA, KATSUHIRA ET AL: "Reaction of 1,4-naphthoquinone with N-methylpyrrole. Selective synthesis of pyrrolylated 1,4-naphthoquinones" CHEM. EXPRESS (1990), 5(10), 749-52 CODEN: CHEXEU;ISSN: 0911-9566, 1990, XP002044652
- MARUYAMA, KAZUHIRO ET AL: "Photochemical reaction of 1,4-naphthoquinone derivatives with pyrrole derivatives. Synthesis of 2-(2-pyrrolyl)-1,4-naphthoquinone derivatives" BULL. CHEM. SOC. JPN. (1985), 58(10), 3049-50 CODEN: BCSJA8;ISSN: 0009-2673, 1985, XP002044653
- ITAHARA, TOSHIO: "Oxidative coupling of quinones and aromatic compounds by palladium(II) acetate" J. ORG. CHEM. (1985), 50(26), 5546-50 CODEN: JOCEAH;ISSN: 0022-3263, 1985, XP002044654
- GAZETTA CHIM. ITAL., vol. 66, pages 215-221, XP002044655

## Description

L'invention concerne l'utilisation de dérivés pyrroliques de la 1,4-naphtoquinone et du 1,4-dihydroxynaphtalène, à titre de colorants directs dans une composition tinctoriale destinée à la teinture des matières kératiniques, notamment dans une composition cosmétique destinée à la teinture des matières kératiniques humaines, et en particulier des fibres kératiniques humaines telle que les cheveux; elle concerne également certains de ces dérivés pyrroliques de la 1,4-naphtoquinone et du 1,4-dihydroxynaphtalène à titre de composés nouveaux.

K. YOSHIDA et Coll., dans la revue Chemistry Express 1990, Vol.5, N°10, pages 749-752, ont étudié la réaction de la 1,4-naphtoquinone sur le N-méthylpyrrole; ils obtinrent par synthèse sélective deux produits colorés : les 2-(1-méthyl-2-pyrrolyl)-1,4-naphtoquinone et 2,5-bis-(1,4-naphtoquinone-2-yl)-pyrrole, utilisables à titre de matières colorantes.

La chimie des pyrrolo-1,4-naphtoquinones n'a cependant été que très peu relatée dans la littérature.

Ainsi, certains dérivés pyrroliques de la 1,4-naphtoquinone sont connus en tant que tels; de tels composés, comme ceux répondant aux formules (a), (b), (c), (d) et (e) suivantes ont été décrits dès 1936 par P. PRATESI dans la publication Gazz. Chim. Ital., Vol.66, pages 215-221, et le Beilstein Reg. Nos. 260480-246571-219671-21973-253088:

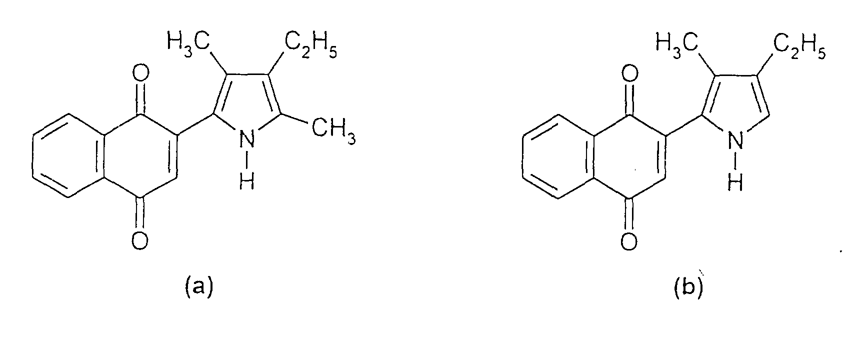

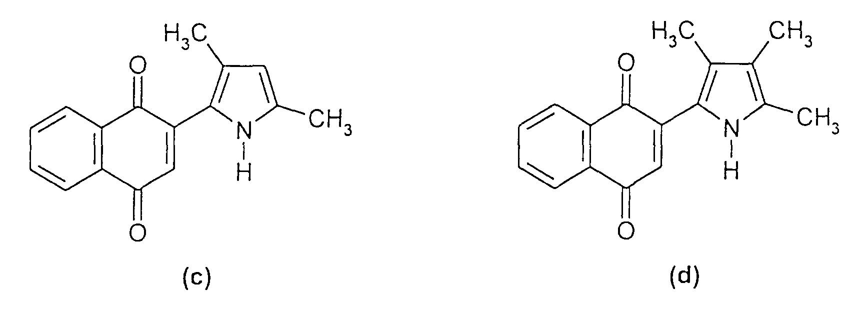

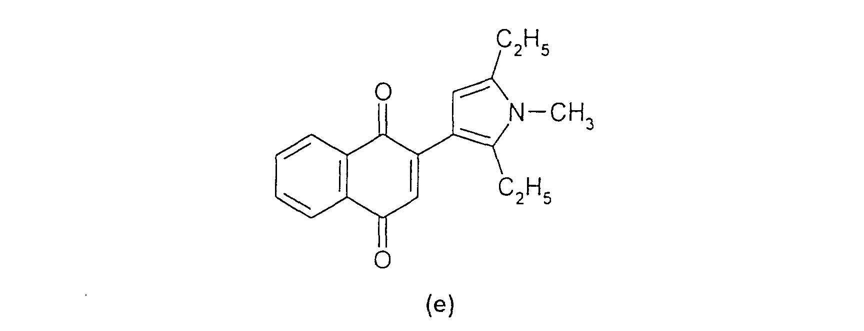

D'autres, synthétisés par voie photochimique, et répondant aux formules (f) et (g) suivantes, ont été décrits par K. MARUYAMA et Coll., dans la revue Bull. Chem. Soc. Jpn., 1985, Vol.58, pages 3049-3050 : 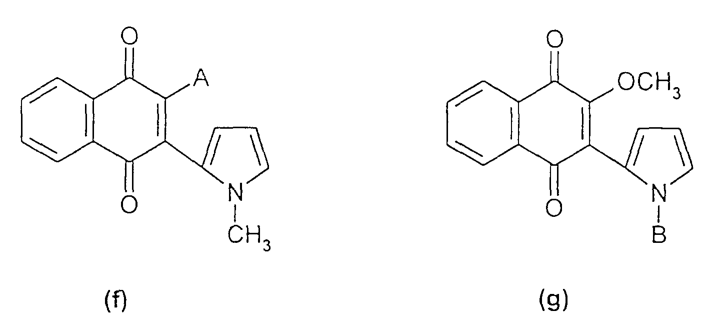 formules (f) et (g) dans lesquelles A désigne H, CH₃, OCH₃, Br,
et B désigne H, CH₃, C₂H₅, CH₂-C₆H₅.

Puis d'autres dérivés pyrroliques de la 1,4-naphtoquinone, connus en tant que tels, ont été synthétisés.

Ainsi, T. ITAHARA et Coll., dans la revue J. Org. Chem. 1985, Vol.50, pages 5546-5550, ont décrit la réaction de la 1,4-naphtoquinone sur le 1-(benzènesulfonyl)-pyrrole dans l'acide acétique à reflux, en présence d'acétate de palladium, pour former le composé de formule (h) suivante :

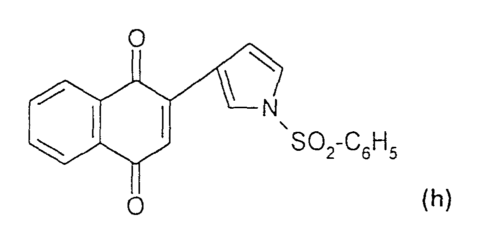

M. MATSUOKA, dans la revue Dyes and Pigments 1991, Vol.16, pages 309-315, a décrit et préparé le composé de formule (i) suivante : 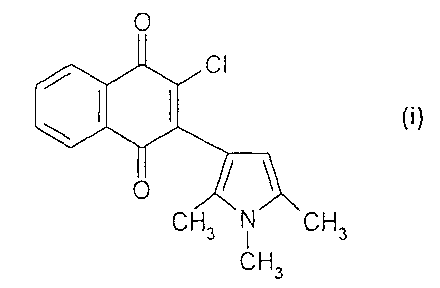 par réaction de la 2,3-dichloro-1,4-naphtoquinone sur le 1,2,5-triméthyl-3-pyrrole, en présence d'alumine acide.

S. YOSHIDA et Coll., dans la revue Chemistry Letters 1996, Vol.2, pages 139-140, ont décrit et préparé le composé de formule (k) suivante : 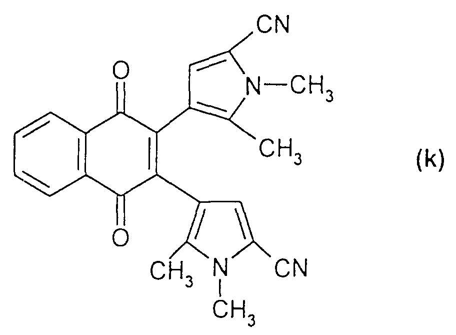 par réaction de la 2,3-dibromo-1,4-naphtoquinone sur le 2-cyano-1,5-diméthyl-4-tributylstannylpyrrole, en présence d'un catalyseur au Palladium.

Parmi les pyrrolo-1,4-dihydroxy-naphtalènes, on ne connaît que le 2-(1-méthyl-2-pyrrolyl)-1,4-dihydroxy-naphtalène qui a été divulgué comme intermédiaire chimique dans la préparation de la 2-(1-méthyl-2-pyrrolyl)-1,4-naphtoquinone dans la revue Chemistry Express 1990 - Vol.5, N°10, pages 750-751, déjà citée ci-dessus.

Or, dans le domaine de la teinture capillaire, on recherche des colorants directs, c'est-à-dire des colorants qui, sans l'apport d'agent oxydant autre que l'air ambiant, sont capables de modifier par eux-mêmes la nuance naturelle des cheveux, de façon temporaire. Dans cette application, les colorants doivent satisfaire à un certain nombre de critères, et notamment engendrer des colorations reproductibles avec des nuances riches et variées permettant d'obtenir une large palette de couleurs susceptible de satisfaire le formulateur.

Parmi les colorants directs classiquement utilisés en teinture capillaire, on connait déjà des colorants naphtoquinoniques tels que la 2-hydroxynaphtoquinone (communément appelée lawsone), la 5-hydroxynaphtoquinone (communément appelée juglone), des 2-hydroxynaphtoquinones et 5-hydroxynaphtoquinones substituées par d'autres radicaux hydroxy, des atomes de chlore, des radicaux alcoxy ou alkyles, telles que décrites dans les brevets FR-2 517 199 et FR-2 537 433.

Cependant lesdits colorants ne permettent pas encore d'obtenir une palette suffisamment riche en nuances variées.

Or, après d'importantes recherches menées sur la question, la Demanderesse vient maintenant de découvrir une nouvelle classe de colorants naphtoquinoniques, des dérivés pyrroliques de la 1,4-naphtoquinone et du 1,4-dihydroxynaphtalène, dont certains sont nouveaux, qui permettent de teindre sans oxydant les matières kératiniques, notamment les matières kératiniques humaines, et en particulier les fibres kératiniques humaines telles que les cheveux. En outre, les teintures capillaires obtenues à l'aide de ces colorants présentent des nuances reproductibles, puissantes et variées.

Cette découverte est à la base de la présente invention.

La présente invention a ainsi pour objet l'utilisation, à titre de colorants directs, dans une composition destinée à la teinture des matières kératiniques, en particulier une composition destinée à la teinture des matières kératiniques humaines, et en particulier des fibres kératiniques humaines telles que les cheveux, de dérivés pyrroliques de la 1,4-naphtoquinone et du 1,4-dihydroxynaphtalène, caractérisés par le fait qu'ils répondent à l'une des formules (I) ou (Il) suivantes : 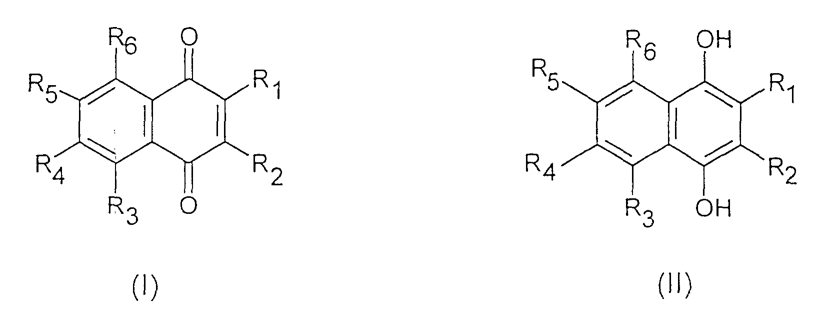 formules (I) ou (Il) dans lesquelles :
- **R**_{**1**} **et R**_{**2**}, identiques ou différents, sont choisis parmi un atome d'hydrogène, un atome d'halogène, un radical OH, un radical alkyle en C₁-C₄, un radical alcoxy en C₁-C₄, ou un radical **Z** de formule (III) définie ci-après,
- **R**_{**3**} **à R**_{**6**}**,** identiques ou différents, sont choisis parmi un atome d'hydrogène, un atome d'halogène, un radical OH, un radical alkyle en C₁-C₄, un radical alcoxy en C₁-C₄,
radical **Z** de formule (III) suivante : 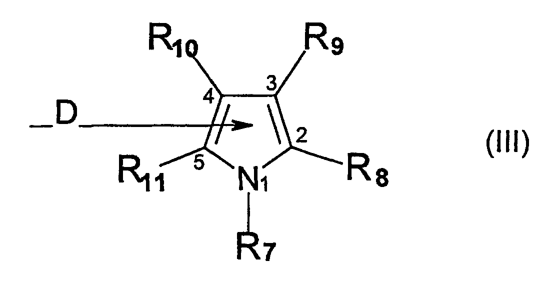 dans laquelle :
- **D** est une liaison covalente entre le noyau pyrrolique (III) et les noyaux de structure (I) ou (II),
- **R**_{**7**} à **R**_{**11**}**,** identiques ou différents, désignent l'hydrogène ou un radical alkyle en C₁-C₁₈ linéaire ou ramifié, saturé ou insaturé, éventuellement substitué par un ou plusieurs atomes d'halogène, par un ou plusieurs radicaux hydroxyles libres ou protégés par une fonction éther ou ester, par un ou plusieurs radicaux -CN, -COOR, -NR'R" et leurs sels d'addition acide, -N(R')COR", -CONR'R", dans lesquels R, R' et R" désignent H ou alkyle (C₁-C₄),
   **R**_{**7**} à **R**_{**11**}**,** identiques ou différents, désignent également un radical CH₂-C₆H₅ ou SO₂-C₆H₅, un radical COalkyle(C₁-C₄), ou COOH et ses sels, ou COOalkyle(C₁-C₄), ou CONR'R" avec R' et R", identiques ou différents, désignant H ou alkyle(C₁-C₄), un radical C₆H₅ qui peut être substitué par un ou plusieurs atomes d'halogène, un ou plusieurs radicaux hydroxy, ou alkyle en C₁-C₄, ou alcoxy en C₁-C₄, et
- **R**_{**8**} à **R**_{**11**} désignent en outre au moins une liaison D,
étant entendu,
- **(i)** qu'au moins un des radicaux R₁ ou R₂ désigne un radical Z,
- **(ii)** que le rattachement de Z aux noyaux de formules (I) ou (II) s'effectue sur l'une quelconque des positions 2 à 5 du noyau pyrrolique,
- **(iii)** et que les radicaux R₈ et R₁₀ ou les radicaux R₉ et R₁₁ ne désignent pas simultanément un composé de formule (I).

Les radicaux alkyle (C₁-C₄) ou les alkyles (C₁-C₄) des radicaux alcoxy ou des radicaux acyles peuvent être linéaires ou ramifiés, saturés ou insaturés, et être choisis notamment au sein des radicaux méthyle, éthyle, n-propyle, isopropyle, n-butyle, isobutyle, ter.-butyle, et plus particulièrement méthyle.

Les radicaux alkyle (C₁-C₁₈) peuvent être linéaires ou ramifiés, saturés ou insaturés; ils peuvent être substitués par un ou plusieurs atomes d'halogène, un ou plusieurs radicaux hydroxyles, libres, ou protégés par une fonction éther choisie parmi les éthers aliphatiques tels que méthoxyméthyléther, méthyléther ou éthyléther, les éthers aromatiques tels que benzyléther, les éthers alkylsilyliques tels que triméthylsilyléther ou tert-butyldiméthylsilyléther, les éthers alkylarylsilyliques tels que tert-butyldiphénylsilyléther ou encore parmi les esters aliphatiques tels qu'acétates et les esters aromatiques tels que benzoates. Ces radicaux alkyle (C₁-C₁₈) peuvent également être substitués par un ou plusieurs radicaux CN, COOR, NR'R" et leurs sels d'addition acide, N(R')COR", CONR'R", dans lesquels R, R' et R" désignent H ou alkyle (C₁-C₄) ci-avant défini.

Les radicaux alkyle (C₁-C₁₈) sont notamment choisis, par exemple, au sein des radicaux méthyle, éthyle, n-propyle, isopropyle, n-butyle, isobutyle, ter.-butyle, n-pentyle, isopentényle, n-octyle, n-hexadécyle, et plus particulièrement méthyle, ter.-butyle, n-pentyle, isopentényle, n-octyle, n-hexadécyle.

Les radicaux de substitution des radicaux alkyle (C₁-C₁₈) qui désignent COOH, ainsi que les radicaux COOH de R₇ à R₁₁ peuvent également être salifiés par des cations non toxiques choisis parmi les alcalins, les alcalino-terreux et ammonium.

Les atomes d'halogène sont choisis notamment parmi le chlore et le brome.

Parmi les dérivés pyrroliques de formules (I) et (II) définies ci-dessus, on peut notamment citer les composés suivants :
― 2-(1,2,5-triméthyl-1-H-pyrrol-4-yl)-3-chloro-[1,4]-naphtoquinone,
― 2-(1,2,5-triméthyl-1-H-pyrrol-4-yl)-[1,4]-naphtoquinone,
― 1-méthyl-2,5-bis-(1,4-naphtoquinone-2-yl)-pyrrole,
― 1-isopentényl-2,5-bis-(1,4-naphtoquinone-2-yl)-pyrrole,
― 2-(4-éthyl-3,5-diméthyl-1H-pyrrol-2-yl)-[1,4]-naphtoquinone,
― 2-(1-benzyl-1-H-pyrrol-2-yl)-3-méthoxy-[1,4]-naphtoquinone,
― 2-(1-méthyl-1-H-pyrrol-2-yl)-3-méthoxy-[1,4]-naphtoquinone,
― 2-(1-éthyl-1-H-pyrrol-2-yl)-3-méthoxy-[1,4]-naphtoquinone,
― 2-(1-H-pyrrol-2-yl)-3-méthoxy-[1,4]-naphtoquinone,
― 2-(1-méthyl-1-H-pyrrol-2-yl)-3-bromo-[1,4]-naphtoquinone,
― 2-(1-méthyl-1-H-pyrrol-2-yl)-3-méthyl-[1,4]-naphtoquinone,
― 2-(1-benzènesulfonyl-1-H-pyrrol-2-yl)-[1,4]-naphtoquinone,
― 2-(2,5-diméthyl-1-H-pyrrol-3-yl)-[1,4]-naphtoquinone,
― 2-(1-pentyl-1H-pyrrol-2-yl)-[1,4]-naphtoquinone,
― 2-(1-octyl-1H-pyrrol-2-yl)-[1,4]-naphtoquinone,
― 2-(1-hexadécyl-1H-pyrrol-2-yl)-[1,4]-naphtoquinone,
― 2-(1-benzyl-1H-pyrrol-2-yl)-[1,4]-naphtoquinone,
― 3-[2-(1,4-dioxo-14-dihydro-naphtalen-2-yl)-pyrrol-1-yl]-propionitrile,
― 1-méthyl-2,5-bis-(1,4-naphtoquinone-2-yl)-pyrrole,
― 1-pentyl-2,5-bis-(1,4-naphtoquinone-2-yl)-pyrrole,
― 1-octyl-2,5-bis-(1,4-naphtoquinone-2-yl)-pyrrole,
― 1-hexadécyl-2,5-bis-(1,4-naphtoquinone-2-yl)-pyrrole,
― 1-benzyl-2,5-bis-(1,4-naphtoquinone-2-yl)-pyrrole,
― ester méthylique de l'acide [5-(1,4-dioxo-1,4-dihydro-naphtalen-2-yl)-1-méthyl-1-H-pyrrol-2-yl]-acétique,
― ester méthylique de l'acide 4-(1,4-dioxo-1,4-dihydro-naphtalen-2-yl)-2,5-diméthyl-1-H-pyrrole-3-carboxylique,
― ester méthylique de l'acide 4-(1,4-dioxo-1,4-dihydro-naphtalen-2-yl)-1,2,5-triméthyl-1-H-pyrrole-3-carboxylique,
― 2-(2,5-diméthyl-1-H-pyrrol-3-yl)-8-hydroxy-[1,4]-naphtoquinone,
― ester méthylique de l'acide 4-(8-hydroxy-1,4-dioxo-1,4-dihydro-naphtalen-2-yl)-1,2,5-triméthyl-1-H-pyrrole-3-carboxylique,
― ester méthylique de l'acide 4-(8-hydroxy-1,4-dioxo-1,4-dihydro-naphtalen-2-yl)-2,5-diméthyl-1-H-pyrrole-3-carboxylique,
― 2-(1-H-pyrrol-2-yl)-[1,4]-naphtoquinone,
― 2-(1-méthyl-1-H-pyrrol-2-yl)-[1,4]-naphtoquinone,
― N-{2-[2-(1,4-dioxo-1,4-dihydro-naphtalen-2-yl)-pyrrol-1-yl]-éthyl }-acétamide,
― 2-{N-[(2-hydroxy-1-hydroxyméthyl-1-méthyl)-éthyl]-1-H-pyrrol-2-yl }-[1,4]-naphtoquinone,
― 2-[N-(β-hydroxyéthyl)-1-H-pyrrol-2-yl]-[1,4]-naphtoquinone,
― acide 2-carboxyméthyl-5-(1,4-dioxo-1,4-dihydro-naphtalen-2-yl)-1,4-diméthyl-1 -H-pyrrole-3-carboxylique,
― acide 4-(1,4-dioxo-1,4-dihydro-naphtalen-2-yl)-2,5-diméthyl-1-H-pyrrole-3-carboxylique,
― ester éthylique de l'acide 4-(1,4-dioxo-1,4-dihydro-naphtalen-2-yl)-3,5-diméthyl-1-H-pyrrole-2-carboxylique,
― 2-(4-acétyl-3,5-diméthyl-1-H-pyrrol-2-yl)- [1,4]-naphtoquinone.
― 2-(1-méthyl-1-H-pyrrol-2-yl)-[1,4]-dihydroxy-naphtalène,
― ester méthylique de l'acide [5-(1,4-dihydroxy-naphtalen-2-yl)-1-méthyl-1-H-pyrrol-2-yl]-acétique.

Parmi les dérivés pyrroliques de la 1,4-naphtoquinone utilisables selon la présente invention, on préfère plus particulièrement les dérivés de formule (I) pour laquelle :
- **R**_{**1**} est un radical **Z** de formule (III) pour laquelle **R**_{**7**} est hydrogène, méthyle, n-pentyle, isopentényle, n-octyle, n-hexadécyle, CH₂-C₆H₅, (CH2)2-CN, (CH₂)₂-OH, (CH₂)₂-NH-COCH₃, C(CH₃)(CH₂OH)₂, **R**_{**8**} est hydrogène, CH₃, COOC₂H₅, CH₂-COOH, CH₂-COOCH₃, 2-(1,4-naphtoquinonyle), 2-(8-hydroxy-1,4-naphtoquinonyle), **R**_{**9**} est hydrogène, CH₃, C₂H₅, COCH₃, COOH ou COOCH₃, **R**_{**10**} est hydrogène, CH₃, 2-(1,4-naphtoquinonyle), ou 2-(8-hydroxy-1,4-naphtoquinonyle), **R**_{**11**} est CH₃ ou 2-(1,4-naphtoquinonyle) ou 2-(8-hydroxy-1,4-naphtoquinonyle),
- **R**_{**2**} est hydrogène,
- **R**_{**3**} à **R**_{**5**} sont hydrogène,
- **R**_{**6**} est hydrogène ou un radical OH.
et plus particulièrement encore les composés suivants :
― 2-(1-H-pyrrol-2-yl)-[1,4]-naphtoquinone,
― 2-(1-méthyl-1H-pyrrol-2-yl)-[1,4]-naphtoquinone,
― 2-(1-pentyl-1H-pyrrol-2-yl)-[1,4]-naphtoquinone,
― 2-(1-octyl-1H-pyrrol-2-yl)-[1,4]-naphtoquinone,
― 2-(1-hexadécyl-1H-pyrrol-2-yl)-[1,4]-naphtoquinone,
― 2-(1-benzyl-1H-pyrrol-2-yl)-[1,4]-naphtoquinone,
― 2-(4-éthyl-3,5-diméthyl-1H-pyrrol-2-yl)-[1,4]-naphtoquinone,
― 3-[2-(1,4-dioxo-1,4-dihydro-naphtalen-2-yl)-pyrrol-1-yl]-propionitrile,
― 1-méthyl-2,5-bis-(1,4-naphtoquinone-2-yl)-pyrrole,
― 1-pentyl-2,5-bis-(1,4-naphtoquinone-2-yl)-pyrrole,
― 1-octyl-2,5-bis-(1,4-naphtoquinone-2-yl)-pyrrole,
― 1-hexadécyl-2,5-bis-(1,4-naphtoquinone-2-yl)-pyrrole,
― 1-benzyl-2,5-bis-(1,4-naphtoquinone-2-yl)-pyrrole,
― ester méthylique de l'acide [5-(1,4-dioxo-1,4-dihydro-naphtalen-2-yl)-1-méthyl-1-H-pyrrol-2-yl]-acétique,
― ester méthylique de l'acide 4-(1,4-dioxo-1,4-dihydro-naphtalen-2-yl)-2,5-diméthyl-1-H-pyrrole-3-carboxylique,
― ester méthylique de l'acide 4-(1,4-dioxo-1,4-dihydro-naphtalen-2-yl)-1,2,5-triméthyl-1-H-pyrrole-3-carboxylique,
― 2-(2,5-diméthyl-1-H-pyrrol-3-yl)-8-hydroxy-[1,4]-naphtoquinone,
― ester méthylique de l'acide 4-(8-hydroxy-1,4-dioxo-1,4-dihydro-naphtalen-2-yl)-1,2,5-triméthyl-1-H-pyrrole-3-carboxylique,
― ester méthylique de l'acide 4-(8-hydroxy-1,4-dioxo-1,4-dihydro-naphtalen-2-yl)-2,5-diméthyl-1-H-pyrrole-3-carboxylique,
― N-{2-[2-(1,4-dioxo-1,4-dihydro-naphtalen-2-yl)-pyrrol-1-yl]-éthyl}-acétamide,
― 2-{N-[(2-hydroxy-1-hydroxyméthyl-1-méthyl)-éthyl]-1-H-pyrrol-2-yl}-[1,4]-naphtoquinone,
― 2-[N-(β-hydroxyéthyl)-1-H-pyrrol-2-yl]-[1,4]-naphtoquinone,
― acide 2-carboxyméthyl-5-(1,4-dioxo-1,4-dihydro-naphtalen-2-yl)-1,4-diméthyl-1-H-pyrrole-3-carboxylique,
― acide 4-(1,4-dioxo-1,4-dihydro-naphtalen-2-yl)-2,5-diméthyl-1-H-pyrrole-3-carboxylique,
― ester éthylique de l'acide 4-(1,4-dioxo-1,4-dihydro-naphtalen-2-yl)-3,5-diméthyl-1-H-pyrrole-2-carboxylique,
― 2-(4-acétyl-3,5-diméthyl-1-H-pyrrol-2-yl)- [1,4]-naphtoquinone.

Parmi les dérivés pyrroliques du 1,4-dihydroxynaphtalène utilisables selon la présente invention, on préfère plus particulièrement le 2-(1-méthyl-1-H-pyrrol-2-yl)-[1,4]-dihydroxy-naphtalène et l'ester méthylique de l'acide [5-(1,4-dihydroxy-naphtalen-2-yl)-1-méthyl-1-H-pyrrol-2-yl]-acétique.

Pour préparer les colorants de formules (I) et (II), on peut procéder classiquement, en suivant le schéma décrit par K. YOSHIDA et Coll., dans l'article Chemistry Express, 1990, Vol.5, N°10, pages 749-752, par réaction de la 1,4-naphtoquinone sur le dérivé pyrrolique [correspondant au colorant de formule (I) ou (II) désiré], et en choisissant un rapport molaire convenable entre les deux réactifs, en milieu solvant et en présence ou non d'un catalyseur; cependant, pour préparer les colorants de formule (II), on travaille toujours en l'absence de catalyseur d'oxydation.
Il est également possible de préparer les composés de formule (I) en effectuant une réaction classique d'oxydation sur les composés de formule (II). De même, il est possible de préparer les composés de formule (II) par une réaction classique de réduction sur les composés de formule (I).

Parmi les composés de formules (I) et (Il) de l'invention, certains sont connus en tant que tels, notamment ceux décrits dans les articles cités précédemment. D'autres sont nouveaux et constituent un autre objet de la présente invention.

La présente invention a ainsi pour objet des composés nouveaux de formule (I) ci-avant définie, comprenant tous les composés de formule (I) à l'exception des suivants :
2-(1-benzènesulfonyl-1-H-pyrrol-2-yl)-[1,4]-naphtoquinone,
2-(1-benzyl-1-H-pyrrol-2-yl)-3-méthoxy-[1,4]-naphtoquinone,
2-(1-éthyl-1-H-pyrrol-2-yl)-3-méthoxy-[1,4]-naphtoquinone,
2-(1-H-pyrrol-2-yl)-3-méthoxy-[1,4]-naphtoquinone,
2-(1-méthyl-1-H-pyrrol-2-yl)-3-bromo-[1,4]-naphtoquinone,
2-(1-méthyl-1-H-pyrrol-2-yl)-3-méthyl-[1,4]-naphtoquinone,
2-(1-méthyl-1 -H-pyrrol-2-yl)-3-méthoxy-[1,4]-naphtoquinone,
2-(1,2,5-triméthyl-1-H-pyrrol-4-yl)-3-chloro-[1,4]-naphtoquinone,
2-(2,5-diéthyl-1-méthyl-pyrrol-3-yl)-[1,4]-naphtoquinone,
1-méthyl-2, 5-bis-(1,4-naphtoquinone-2-yl)-pyrrole,
ainsi que des composés de formule (IV) suivante : 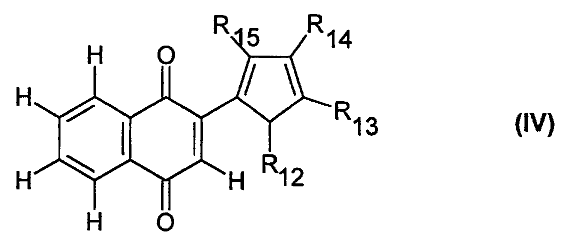 formule dans laquelle R₁₂ désigne H , alkyle, aryle ou aralkyle, et R₁₃, R₁₄, R₁₅, identiques ou différents sont H, alkyle, phényle, sulfonyle substitué par un phényle.

Ces **nouveaux** composés de formule (I) sont plus particulièrement choisis parmi les suivants :
2-(1-H-pyrrol-2-yl)-[1,4]-naphtoquinone,
2-(1-pentyl-1 H-pyrrol-2-yl)-[1,4]-naphtoquinone,
2-(1-octyl-1H-pyrrol-2-yl)-[1,4]-naphtoquinone,
2-(1-hexadécyl-1H-pyrrol-2-yl)-[1,4]-naphtoquinone,
2-(1-benzyl-1 H-pyrrol-2-yl)-[1,4]-naphtoquinone,
2-(1,2,5-triméthyl-1-H-pyrrol-4-yl)-[1,4]-naphtoquinone,
3-[2-(1,4-dioxo-1,4-dihydro-naphtalen-2-yl)-pyrrol-1-yl]-propionitrile,
ester méthylique de l'acide [5-(1,4-dioxo-1,4-dihydro-naphtalen-2-yl)-1-méthyl-1-H-pyrrol-2-yl]-acétique,
ester méthylique de l'acide 4-(1,4-dioxo-1,4-dihydro-naphtalen-2-yl)-2,5-diméthyl-1-H-pyrrole-3-carbaxylique,
ester méthylique de l'acide 4-(1,4-dioxo-1,4-dihydro-naphtalen-2-yl)-1,2,5-triméthyl-1-H-pyrrole-3-carboxylique,
2-(2,5-diméthyl-1-H-pyrrol-3-yl)-8-hydroxy-[1,4]-naphtoquinone,
ester méthylique de l'acide 4-(8-hydroxy-1,4-dioxo-1,4-dihydro-naphtalen-2-yl)-1,2,5-triméthyl-1-H-pyrrole-3-carboxylique,
ester méthylique de l'acide 4-(8-hydroxy-1,4-dioxo-1,4-dihydro-naphtalen-2-yl)-2,5-diméthyl-1-H-pyrrole-3-carboxylique,
2-(2,5-diméthyl-1-H-pyrrol-3-yl)-[1,4]-naphtoquinone,
1-pentyl-2,5-bis-(1,4-naphtoquinone-2-yl)-pyrrole,
1-octyl-2,5-bis-(1,4-naphtoquinone-2-yl)-pyrrole,
1-hexadécyl-2,5-bis-(1,4-naphtoquinone-2-yl)-pyrrole,
1-benzyl-2,5-bis-(1,4-naphtoquinone-2-yl)-pyrrole,
2-[1-(β-hydroxyéthyl)-1H-pyrrol-2-yl]-[1,4]-naphtoquinone,
2-{N-[(2-hydroxy-1-hydroxyméthyl-1-méthyl)-éthyl]-1-H-pyrrol-2-yl }-[1,4]-naphtoquinone,
N-{2-[2-(1,4-dioxo-1,4-dihydro-naphtalen-2-yl)-pyrrol-1-yl]-éthyl}-acétamide,
acide 2-carboxyméthyl-5-(1,4-dioxo-1,4-dihydro-naphtalen-2-yl)-1,4-diméthyl-1H-pyrrole-3-carboxylique,
acide 4-(1,4-dioxo-1,4-dihydro-naphtalen-2-yl)-2,5-diméthyl-1H-pyrrole-3-carboxylique,
ester éthylique de l'acide 4-(1,4-dioxo-1,4-dihydro-naphtalen-2-yl)-3,5-diméthyl-1-H-pyrrole-2-carboxylique,
2-(4-acétyl-3,5-diméthyl-1-H-pyrrol-2-yl]-[1,4]-naphtoquinone.

La présente invention a aussi pour objet des composés **nouveaux** de formule (II) ci-avant définie, comprenant tous les composés de formule (Il) à l'exclusion du composé suivant :
2-(1-méthyl-1-H-pyrrol-2-yl)-[1,4]-dihydroxy-naphtalène.

Un composé **nouveau** de formule (Il) plus particulièrement préféré est l'ester méthylique de l'acide [5-(1,4-dihydroxy-naphtalen-2-yl)-1-méthyl-1-H-pyrrol-2-yl]-acétique.

L'invention a également pour objet une composition tinctoriale pour matières kératiniques, comprenant dans un milieu approprié pour la teinture, 0,01 à 10% en poids par rapport au poids total de la composition, d'au moins un composé de formule (I) ou (II) définie ci-avant.

Elle a aussi pour objet une composition cosmétique destinée à la teinture des matières kératiniques humaines, et plus particulièrement encore, à la teinture des fibres kératiniques humaines telles que les cheveux, comprenant dans un milieu cosmétiquement acceptable, 0,01 à 10% en poids par rapport au poids total de la composition d'au moins un composé de formule (I) ou (Il) définie ci-avant.

Elle a également pour objet les procédés de teinture mettant en oeuvre ces compositions.

Mais d'autres caractéristiques, aspects et avantages de l'invention apparaîtront encore plus clairement à la lecture de la description qui va suivre, ainsi que des divers exemples concrets, mais nullement limitatifs, destinés à l'illustrer.

Au sens de l'invention, on entend principalement par matières kératiniques, les fibres textiles naturelles telles que la laine, la fourrure animale, par matières kératiniques humaines, la peau et les ongles, et par fibres kératiniques humaines, les cheveux, les poils, les cils et les sourcils. L'invention porte plus particulièrement sur les matières kératiniques humaines, et plus particulièrement encore sur les cheveux.

Les composés de formule (I) ou (II) sont généralement présents dans des proportions comprises entre environ 0,01 et 10%, de préférence entre environ 0,05 et 5% en poids, par rapport au poids total de la composition de teinture.

Les composés de formule (I) ou (Il) peuvent également être incorporés dans des compositions tinctoriales pour la coloration d'oxydation qui contiennent des bases d'oxydation et éventuellement des coupleurs, pour enrichir en reflets les nuances obtenues avec les colorants d'oxydation.

Le milieu cosmétiquement acceptable est de préférence un milieu constitué par de l'eau et/ou des solvants organiques acceptables sur le plan cosmétique si la composition est cosmétique, et plus particulièrement, des alcools (l'alcool éthylique, l'alcool isopropylique, l'alcool benzylique, et l'alcool phényléthylique), des glycols ou éthers de glycol (le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, les éthers monométhylique, monoéthylique et monobutylique d'éthylèneglycol), dans des concentrations comprises entre environ 0,5 et 25% et, de préférence, entre environ 2 et 15% en poids par rapport au poids total de la composition.
Il peut aussi contenir des corps gras tels que des huiles et des cires.
On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.

La composition tinctoriale selon l'invention peut encore contenir, pour obtenir des teintes variées, outre les colorants de formule (I) ou (Il), d'autre(s) colorant(s) direct(s) classiquement utilisés, et notamment, des colorants nitrés benzéniques, comme les nitrophénylènediamines, les nitrodiphénylamines, les nitroanilines, les éthers de phénol nitrés ou les nitrophénols, des nitropyridines, des colorants anthraquinoniques, mono- ou di-azoïques, triarylméthaniques, aziniques, acridiniques et xanthéniques, ou encore des colorants métallifères.
La proportion de tous ces autres colorants directs d'addition peut varier entre environ 0,05 et 10 % en poids par rapport au poids total de la composition tinctoriale.

Ladite composition tinctoriale peut contenir en outre tout autre adjuvant utilisé habituellement en teinture des matières kératiniques et par exemple, des agents tensio-actifs bien connus de l'état de la technique et de type anionique, cationique, non-ionique, amphotère, zwittérionique ou leurs mélanges, des agents épaississants, des agents anti-oxydants, des parfums, des agents séquestrants, des agents dispersants, des agents de conditionnement, des agents conservateurs, des agents opacifiants, etc...
Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition de teinture selon l'invention peut être formulée à pH acide, neutre ou alcalin, le pH pouvant varier par exemple de 2 à 11 et de préférence de 2,5 à 10, et pouvant être ajusté au moyen d'agents d'alcalinisation ou d'agents d'acidification antérieurement bien connus.
Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 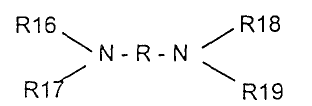 dans laquelle R est un reste propylène éventuellement substitué par un groupement hydroxyle ou un radical alkyle en C₁-C₄ ; R₁₆, R₁₇, R₁₈ et R₁₉, identiques ou différents, représentent un atome d'hydrogène, un radical alkyle en C₁-C₄ ou hydroxyalkyle en C₁-C₄.
Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Lorsque la composition est destinée à être appliquée sur les fibres kératiniques humaines, elle peut se présenter sous des formes diverses, telles que sous forme de liquide, de crème, de gel ou sous toute autre forme appropriée pour réaliser une teinture des fibres kératiniques. En particulier, elle peut être conditionnée sous pression en flacon aérosol en présence d'un agent propulseur et former une mousse.
Les compositions pour les ongles et la peau sont notamment des vernis à ongles, des produits de maquillage pour les lèvres ou le visage, tels que les bâtons de rouge à lèvres, les fards à paupière, les fards à joue, les fonds de teint, les ligneurs encore appelés "eye-liner", ou les mascaras.

Un autre objet de l'invention porte sur un procédé de teinture des fibres kératiniques humaines, et plus particulièrement des cheveux, par coloration directe, consistant à laisser agir une composition tinctoriale renfermant au moins un composé de formule (I) ou (II), sur les fibres kératiniques sèches ou humides. On peut utiliser la composition selon l'invention en tant que composition non rincée, c'est-à-dire qu'après application de la composition sur les fibres, on sèche sans rinçage intermédiaire. Dans les autres modes d'application, on laisse agir la composition sur les fibres pendant un temps de pose variant entre 3 et 60 minutes environ, de préférence entre 5 et 45 minutes environ, on rince, éventuellement on lave et on rince à nouveau, puis on sèche.

On va maintenant donner, à titre d'illustration, et sans aucun caractère limitatif, plusieurs exemples de préparation de composés de formule (I) et (Il) selon l'invention, ainsi que des exemples concrets de teinture à base de tels composés.

### EXEMPLES DE PREPARATION

### EXEMPLE 1 :

### Préparation de la 2-(1-méthyl-1-H-pyrrol-2-yl)-[1,4]-naphtoquinone

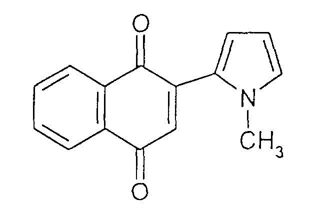

Selon le protocole décrit dans l'article Chemistry Express 1990, Vol.5, N°10, pages 749-752, on a agité dans un ballon, 3,16 millimoles de 1,4-naphtoquinone avec 25 millimoles de N-méthyl-pyrrole et 3,16 millimoles d'acétate de cuivre dans 50 ml d'acide acétique à température ambiante pendant 1 heure. On a dilué le mélange réactionnel par 300 ml de dichlorométhane et versé ensuite le tout dans un grand volume d'eau (300 ml). On a ensuite séparé la phase organique, qu'on a lavée à quatre reprises par 100 ml d'eau, et en dernier lieu par une solution aqueuse saturée de NaCI. On a séché sur Na₂SO₄ puis on a concentré sous vide. On a ainsi isolé le produit 2-(1-méthyl-1-H-pyrrol-2-yl)-[1,4]-naphtoquinone avec un rendement de 79% après purification par chromatographie sur colonne de silice.
**RMN** ^{**1**}**H 200MHz** (DMSOd6, δ ppm) : 3.68 (s, 3H), 6.18 (d.d, 1H), 6.61 (d.d, 1H), 6.95 (s, 1H), 7.11 (m, 1H), 7.8 à 8.1 (m, 4H).
**Point de Fusion** : 95-96°C.

### EXEMPLE 2 :

### Préparation de la 2-(1-pentyl-1 -H-pyrrol-2-yl)-[1,4]-naphtoquinone

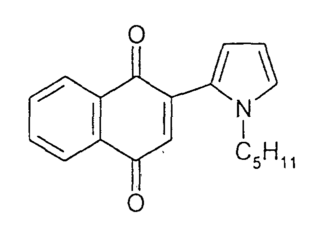

On a agité dans un ballon 6,32 millimoles de 1,4-naphtoquinone et 3,16 millimoles de N-pentyl-pyrrole dans 50 ml de chloroforme, pendant 5 heures à température ambiante en catalysant la réaction par addition de quelques milligrammes d'acide para-toluène sulfonique. On a dilué le milieu réactionnel par 50 ml de chloroforme, puis on a lavé à quatre reprises par 50 ml d'eau et en dernier lieu par une solution aqueuse saturée de NaCI. On a séché sur Na₂SO₄ puis on a concentré sous vide. On a ainsi isolé le produit 2-(1-pentyl-1-H-pyrrol-2-yl)-[1,4]-naphtoquinone, sous forme d'huile, avec un rendement de 26% après purification par chromatographie sur colonne de silice.
**RMN** ^{**1**}**H 200MHz** (CDCl₃, δ ppm) : 0.85 (t, 3H), 1.16 à 1.34 (m, 4H), 1.65 à 1.80 (m, 2H), 3.95 (t, 2H), 6.28 (d.d, 1H), 6.60 (d.d, 1H), 6.88 (s, 1H), 6.97 (m, 1H), 7.72 à 7.81 (m, 2H), 8.09 à 8.19 (m, 2H).

### EXEMPLE 3 :

### Préparation de la 2-(1-octyl-1-H-pyrrol-2-yl)-[1,4]-naphtoquinone

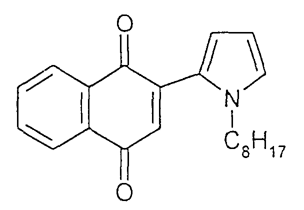

On a agité dans un ballon 6,32 millimoles de 1,4-naphtoquinone et 3,16 millimoles de N-octyl-pyrrole dans 50 ml de chloroforme, pendant 5 heures à température ambiante en catalysant la réaction par addition de quelques milligrammes d'acide para-toluène sulfonique. On a dilué le milieu réactionnel par 50 ml de chloroforme, puis on a lavé à quatre reprises par 50 ml d'eau et en dernier lieu par une solution aqueuse saturée de NaCI. On a séché sur Na₂SO₄ puis on a concentré sous vide. On a ainsi isolé le produit 2-(1-octyl-1-H-pyrrol-2-yl)-[1,4]-naphtoquinone, sous forme d'huile, avec un rendement de 32% après purification par chromatographie sur colonne de silice.

### EXEMPLE 4 :

### Préparation de la 2-(1-hexadécyl-1-H-pyrrol-2-yl)-[1,4]-naphtoquinone

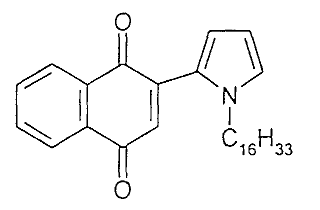

On a agité dans un ballon 6,32 millimoles de 1,4-naphtoquinone et 3,16 millimoles de N-hexadécyl-pyrrole dans 50 ml de chloroforme, pendant 5 heures à température ambiante en catalysant la réaction par addition de quelques milligrammes d'acide para-toluène sulfonique. On a dilué le milieu réactionnel par 50 ml de chloroforme, puis on a lavé à quatre reprises par 50 ml d'eau et en dernier lieu par une solution aqueuse saturée de NaCI. On a séché sur Na₂SO₄ puis on a concentré sous vide. On a ainsi isolé le produit 2-(1-hexadecyl-1-H-pyrrol-2-yl)-[1,4]-naphtoquinone avec un rendement de 41% après purification par chromatographie sur colonne de silice.
**RMN** ^{**1**}**H 200MHz** (CDCl₃, δ ppm) : 0.9 (t, 3H), 1.05 à 1.45 (m, 26H), 1.75 (m, 2H), 3.94 (t, 2H), 6.28 (d.d, 1H), 6.59 (d.d, 1H), 6.88 (s, 1H), 6.96 (m, 1H), 7.74 à 7.80 (m, 2H), 8.10 à 8.19 (m, 2H).
Point de Fusion : 56-57°C.

### EXEMPLE 5 :

### Préparation de la 2 - (1-benzyl-1-H-pyrrol-2-yl)-[1,4]-naphtoquinone

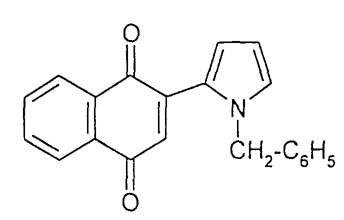

On a agité dans un ballon, 3,16 millimoles de 1,4-naphtoquinone avec 25 millimoles de N-benzyl-pyrrole et 3,16 millimoles d'acétate de cuivre dans 50 ml d'acide acétique à température ambiante pendant 1 heure. On a dilué le mélange réactionnel par 300 ml de dichlorométhane et versé ensuite le tout dans un grand volume d'eau (300 ml). On a ensuite séparé la phase organique, qu'on a lavée à quatre reprises par 100 ml d'eau, et en dernier lieu par une solution aqueuse saturée de NaCI. On a séché sur Na₂SO₄ puis on a concentré sous vide. On a ainsi isolé le produit 2-(1-benzyl-1-H-pyrrol-2-yl)-[1,4]-naphtoquinone avec un rendement de 18% après purification par chromatographie sur colonne de silice.
**Point de Fusion :** 99°C.

### EXEMPLE 6 :

### Préparation du 3-[2-(1,4-dioxo-1,4-dihydro-naphtalen-2-yl)-pyrrol-1-yl]-propionitrile

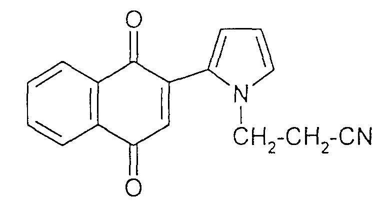

On a agité dans un ballon 6,32 millimoles de 1,4-naphtoquinone et 3,16 millimoles de 3-(pyrrol-1-yl)-propionitrile dans 50 ml de chloroforme, pendant 5 heures à température ambiante en catalysant la réaction par addition de quelques milligrammes d'acide para-toluène sulfonique. On a dilué le milieu réactionnel par 50 ml de chloroforme, puis on a lavé à quatre reprises par 50 ml d'eau et en dernier lieu par une solution aqueuse saturée de NaCI. On a séché sur Na₂SO₄ puis on a concentré sous vide. On a ainsi isolé le produit 3-[2-(1,4-dioxo-1,4-dihydro-naphtalen-2-yl)-pyrrol-1-yl]-propionitrile avec un rendement de 42% après purification par chromatographie sur colonne de silice.
**Point de Fusion :** 127-128°C.

### EXEMPLE 7 :

### Préparation du 1-méthyl -2,5 -bis -(1,4-naphtoquinone-2 -yl )-pyrrole

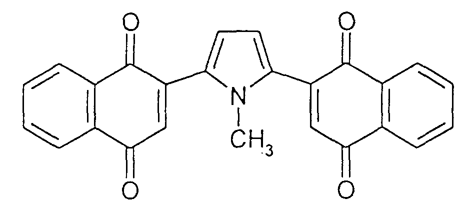

On a suivi le protocole décrit dans l'article Chemistry Express 1990, Vol.5, N°10, pages 749-752, en agitant 10 millimoles de 1,4-naphtoquinone avec 2,5 millimoles de N-méthyl-pyrrole dans 40ml d'acide acétique à 50°C pendant 5 heures. On a dilué le mélange réactionnel par 300 ml de dichlorométhane et versé ensuite le tout dans un grand volume d'eau (300 ml). On a ensuite séparé la phase organique, qu'on a lavée à quatre reprises par 100 ml d'eau, et en dernier lieu par une solution aqueuse saturée de NaCI. On a séché sur Na₂SO₄ puis on a concentré sous vide. On a ainsi obtenu le 1-méthyl-2,5-bis-(1,4-naphtoquinone-2-yl)-pyrrole avec un rendement de 90%.
**Point de Fusion** : 320-321°C.

### EXEMPLE 8 :

### Préparation du 1-pentyl-2,5-bis-(1,4-naphtoquinone-2-yl)-pyrrole

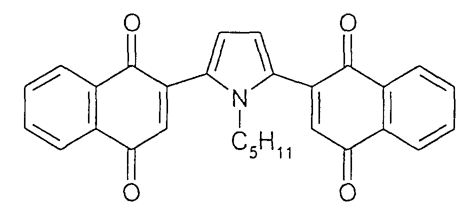

On a suivi le même protocole qu'à l'exemple 7 en agitant 10 millimoles de 1,4-naphtoquinone avec 2,5 millimoles de N-pentyl-pyrrole dans 40ml d'acide acétique à 50°C pendant 5 heures.

On a ainsi obtenu le 1-pentyl-2,5-bis-(1,4-naphtoquinone-2-yl)-pyrrole avec un rendement de 87% après chromatographie sur colonne de silice.
**RMN** ^{**1**}**H 200MHz** (CDCl₃, δ ppm) : 0.62 (t, 3H), 0.8 à 1,4 (m, 6H), 4.0 (t, 2H), 6.63 (s, 2H), 7.06 (s, 2H), 7.75 à 7.85 (m, 4H), 8.06 à 8.23 (m, 4H).
**Point de Fusion :** 128°C.

### EXEMPLE 9 :

### Préparation du 1-octyl-2,5-bis-(1,4-naphtoquinone-2-yl)-pyrrole

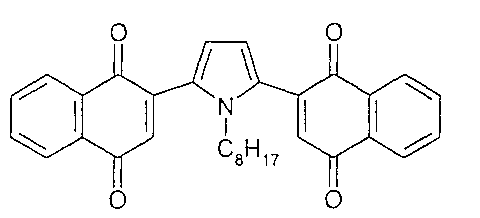

On a suivi le même protocole qu'à l'exemple 7 en agitant 10 millimoles de 1,4-naphtoquinone avec 2,5 millimoles de N-octyl-pyrrole dans 40ml d'acide acétique à 50°C pendant 5 heures.
On a ainsi obtenu le 1-octyl-2,5-bis-(1,4-naphtoquinone-2-yl)-pyrrole avec un rendement de 49% après chromatographie sur colonne de silice.
**Point de Fusion :** 75-76°C.

### EXEMPLE 10 :

### Préparation du 1-hexadécyl-2,5-bis-(1,4-naphtoquinone-2-yl)-pyrrole

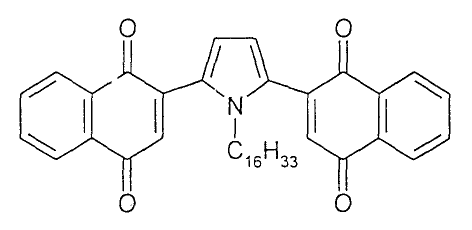

On a suivi le même protocole qu'à l'exemple 7 en agitant 10 millimoles de 1,4-naphtoquinone avec 2,5 millimoles de N-hexadécyl-pyrrole dans 40ml d'acide acétique à 50°C pendant 5 heures.

On a ainsi obtenu le 1-hexadécyl-2,5-bis-(1,4-naphtoquinone-2-yl)-pyrrole avec un rendement de 38% après chromatographie sur colonne de silice.
**RMN** ^{**1**}**H 200MHz** (CDCl₃, δ ppm) : 0.8 à 1.5 (m, 31H), 4.00 (t, 2H), 6.63 (s, 2H), 7.05 (s, 2H), 7.74 à 7.85 (m, 4H), 8.06 à 8.23 (m, 4H).
**Point de Fusion** : 40-43°C.

### EXEMPLE 11 :

### Préparation du 1-benzyl-2,5-bis-(1,4-naphtoquinone-2-yl)-pyrrole

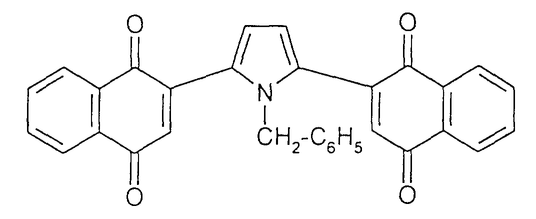

On a suivi le même protocole qu'à l'exemple 7 en agitant 10 millimoles de 1,4-naphtoquinone avec 2,5 millimoles de N-benzyl-pyrrole dans 40ml d'acide acétique à 50°C pendant 5 heures.
On a ainsi obtenu le 1-benzyl-2,5-bis-(1,4-naphtoquinone-2-yl)-pyrrole avec un rendement de 9% après chromatographie sur colonne de silice.
**Point de Fusion** : 149-150°C.

### EXEMPLE 12 :

### Préparation de l'ester méthylique de l'acide [5-(1,4-dihydroxy-naphtalen-2-yl)-1-méthyl-1-H-pyrrol-2-yl]-acétique.

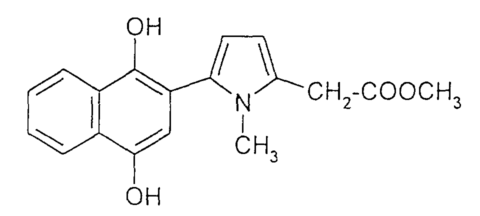

On a agité dans un ballon 40 millimoles de 1,4-naphtoquinone et 10 millimoles de l'ester méthylique de l'acide 2-(N-méthyl-1-H-pyrrol)-acétique dans 50 ml d'acide acétique glacial pendant 2 heures à température ambiante. On a dilué le mélange réactionnel par 300 ml de chloroforme et versé ensuite le tout dans un grand volume d'eau (300 ml). On a ensuite séparé la phase organique, qu'on a lavée à quatre reprises par 100 ml d'eau, et en dernier lieu par une solution aqueuse saturée de NaCI. On a séché sur Na₂SO₄ puis on a concentré sous vide. On a ainsi isolé le produit ester méthylique de l'acide [5-(1,4-dihydroxy-naphtalen-2-yl)-1-méthyl-1-H-pyrrol-2-yl]-acétique, avec un rendement de 12% après purification par chromatographie sur colonne de silice.
**RMN** ^{**1**}**H 200MHz** (DMSO d6 δ ppm) : 3.35 (s, 3H), 3.66 (s, 3H), 3.86 (s, 2H), 5.97 (d, 1H), 6.02 (d, 1H), 6.64 (s, 1H), 7.41 à 7.52 (m, 2H), 8.05 à 8.17 (m, 2H), 8.45 (s, 1H), 9.56 (s, 1H).
**Point de Fusion :** 200-201°C.

### EXEMPLE 13 :

### Préparation de l'ester méthylique de l'acide [5-(1,4-dioxo-1,4-dihydro-naphtalen-2-yl)-1-méthyl-1-H-pyrrol-2-yl]-acétique.

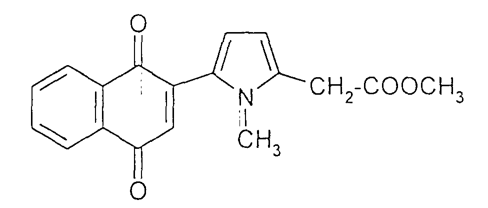

En procédant comme à l'exemple 12, on a également isolé par chromatographie sur colonne de silice, le produit ester méthylique de l'acide [5-(1,4-dioxo-1,4-dihydro-naphtalen-2-yl)-1-méthyl-1-H-pyrrol-2-yl]-acétique, avec un rendement de 56%.
**RMN** ^{**1**}**H 200MHz** (DMSO d6 δ ppm) : 3.50 (s, 3H), 3.66 (s, 3H), 3.85 (s, 2H), 6.13 (d, 1H), 6.49 (d, 1H), 6.86 (s, 1H), 7.82 à 7.91 (m, 2H), 7.95 à 8.08 (m, 2H).
Point de Fusion : 89-90°C.

### EXEMPLE 14 :

### Préparation de l'ester méthylique de l'acide 4-(1,4-dioxo-1,4-dihydro-naphtalen-2-yl) -2,5 -diméthyl-1-H-pyrrole-3 -carboxylique.

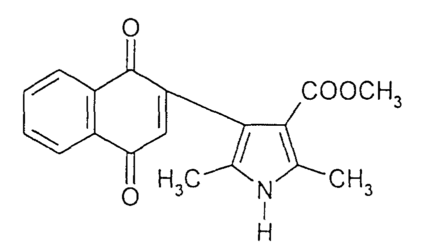

On a agité dans un ballon 40 millimoles de 1,4-naphtoquinone et 10 millimoles de l'ester méthylique de l'acide 3-(2,5-diméthyl-1-H-pyrrole) -carboxylique dans 50 ml d'acide acétique glacial pendant 2 heures à température ambiante. On a dilué le mélange réactionnel par 300 ml de chloroforme et versé ensuite le tout dans un grand volume d'eau (300 ml). On a ensuite séparé la phase organique, qu'on a lavée à quatre reprises par 100 ml d'eau, et en dernier lieu par une solution aqueuse saturée de NaCI. On a séché sur Na₂SO₄ puis on a concentré sous vide. On a ainsi isolé le produit ester méthylique de l'acide 4-(1,4-dioxo-1,4-dihydro-naphtalen-2-yl)-2,5-diméthyl-1-H-pyrrole-3-carboxylique, avec un rendement de 39% après purification par chromatographie sur colonne de silice.
**RMN** ^{**1**}**H 200MHz** (DMSO d6 δ ppm) : 2.17 (s, 3H), 2.38 (s, 3H), 3.44 (s, 3H), 6.77 (s, 1H), 7.85 à 7.91 (m, 2H), 7.97 à 8.04 (m, 2H), 11.44 (s élargi, 1H).
**Point de Fusion** : 209-210°C.

### EXEMPLE 15 :

### Préparation de la 2-(2,5-diméthyl-1-H-pyrrol-3-yl)-8-hydroxy-[1,4]-naphtoquinone,

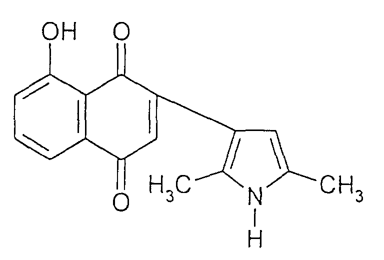

On a suivi le même protocole qu'à l'exemple 14 en agitant dans un ballon 40 millimoles de 1,4-naphtoquinone et 10 millimoles de 2,5-diméthyl-1H-pyrrole dans 50 ml d'acide acétique glacial pendant 2 heures à température ambiante.
On a ainsi isolé le produit 2-(2,5-diméthyl-1-H-pyrrol-3-yl)-8-hydroxy-[1,4]-naphtoquinone, avec un rendement de 37% après purification par chromatographie sur colonne de silice.
**RMN** ^{**1**}**H 200MHz** (DMSO d6 δ ppm) : 2.15 (s, 3H), 2.30 (s, 3H), 6.14 (m, 1H), 6.68 (s, 1H), 7.30 (d.d, 1H), 7.48 (d.d, 1H), 7.73 (d.d, 1H), 11.0 (s élargi, 1H), 12.11 (s, 1H).
**Point de Fusion :** 195-196°C.

### EXEMPLE 16 :

### Préparation de l'ester méthylique de l'acide 4 - (8 -hvdroxv -1,4 -dioxo -1,4 -dihydro-naphtalen-2-yl)-1,2,5-triméthyl-1-H-pyrrole-3-carboxylique.

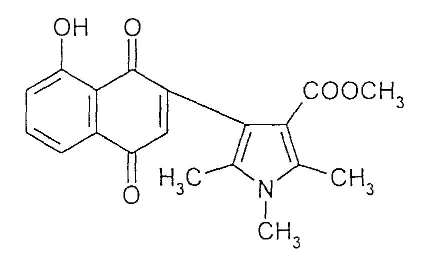

On a suivi le même protocole qu'à l'exemple 14 en agitant dans un ballon 40 millimoles de 1,4-naphtoquinone et 10 millimoles de l'ester méthylique de l'acide 3-(1,2,5-triméthyl-1-H-pyrrole)-carboxylique dans 50 ml d'acide acétique glacial pendant 2 heures à température ambiante.
On a ainsi isolé le produit ester méthylique de l'acide 4-(8-hydroxy-1,4-dioxo-1,4-dihydro-naphtalen-2-yl)-1,2,5-triméthyl-1-H-pyrrole-3-carboxylique, avec un rendement de 26% après purification par chromatographie sur colonne de silice.
**RMN** ^{**1**}**H 200MHz** (DMSO d6 δ ppm) : 2.19 (s, 3H), 2.47 (s, 3H), 3.46 (s, 3H), 3.48 (s, 3H), 6.74 (s, 1H), 7.36 (d.d, 1H), 7.55 (d.d, 1H), 7.78 (d.d, 1H), 11.9 (s, 1H).
**Point de Fusion** : 178-180°C avec décomposition.

### EXEMPLE 17 :

### Préparation de l'ester méthylique de l'acide 4 - (8-hydroxy-1,4 -dioxo -1,4-dihydro-naphtalen-2-yl)-,2,5-diméthyl-1 -H-pyrrole-3-carboxylique.

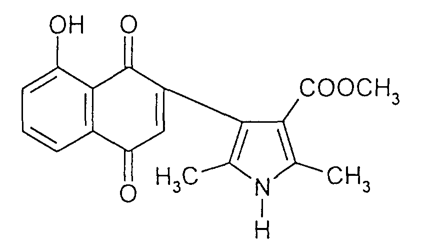

On a suivi le même protocole qu'à l'exemple 14 en agitant dans un ballon 40 millimoles de 1,4-naphtoquinone et 10 millimoles de l'ester méthylique de l'acide 3-(2,5-diméthyl-1-H-pyrrole)-carboxylique dans 50 ml d'acide acétique glacial pendant 2 heures à température ambiante.
On a ainsi isolé le produit ester méthylique de l'acide 4-(8-hydroxy-1,4-dioxo-1,4-dihydro-naphtalen-2-yl)-2,5-diméthyl-1-H-pyrrole-3-carboxylique, avec un rendement de 31% après purification par chromatographie sur colonne de silice.
**RMN** ^{**1**}**H 200MHz** (DMSO d6 δ ppm) : 2.17 (s, 3H), 2.39 (s, 3H), 3.48 (s, 3H), 6.77 (s, 1H), 7.36 (d.d, 1H), 7.55 (d.d, 1H), 7.78 (d.d, 1H), 11.47 (s élargi, 1H), 11.91 (s, 1H).
**Point de Fusion** : 98-99°C avec décomposition.

### EXEMPLE 18 :

### Préparation de la 2-(1-H-pyrrol-2-yl)-[1,4]-naphtoquinone

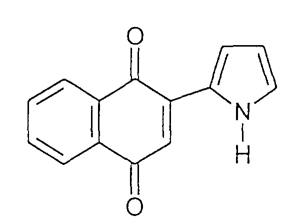

En utilisant le protocole décrit à l'exemple 1, à partir de 3,16 millimoles de 1,4-naphtoquinone, 25 millimoles de pyrrole, et 3,16 millimoles d'acétate de cuivre dans 50 ml d'acide acétique, on a isolé le produit 2-(1H-pyrrol-2-yl)-[1,4]-naphtoquinone avec un rendement de 18% après purification par chromatographie sur colonne de silice.
**Point de Fusion** : 155-158°C.

### EXEMPLE 19 :

### Préparation de la 2-(4-éthyl-3,5-diméthyl-1-H-pyrrol-2-yl)-[1,4]-naphtoquinone

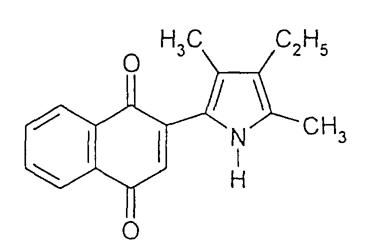

On a agité dans un ballon 6,32 millimoles de 1,4-naphtoquinone et 3,16 millimoles de 2,4-diméthyl-3-éthyl-pyrrole dans 20 ml d'acide acétique glacial pendant 10 minutes à température ambiante. On a dilué le mélange réactionnel par 300 ml de dichlorométhane et versé ensuite le tout dans un grand volume d'eau (300 ml). On a ensuite séparé la phase organique, qu'on a lavée à quatre reprises par 100 ml d'eau, et en dernier lieu par une solution aqueuse saturée de NaCI. On a séché sur Na₂SO₄ puis on a concentré sous vide. On a ainsi isolé le produit 2-(4-éthyl-3,5-diméthyl-1-H-pyrrol-2-yl)-[1,4]-naphtoquinone, avec un rendement de 80% après purification par chromatographie sur colonne de silice.
**RMN** ^{**1**}**H 200MHz** (CDCl₃ , δ ppm) : 0.98 (t, 3H), 2.17 (s, 6H), 2.26 (q, 2H), 6.77 (s, 1H), 7.5 à 7.65 (m, 2H), 7.85 à 8.05 (m, 2H), 10.49 (s élargi, 1H).

### EXEMPLES DE COMPOSITIONS TINCTORIALES

### EXEMPLE 20 :

On a préparé la composition de teinture suivante :

| | |
|---|---|
| Colorant préparé à l'exemple 1 | 0,531g |
| Alcool benzylique | 10,0 g |
| Alcool éthylique | 21,0 g |
| Glycérine | 5,0 g |
| Hydroxyéthylcellulose vendue sous la dénomination Cellosize QP4400H par la société Union Carbide | 2,3 g |
| Acide citrique | 1,4 g |
| Eau déminéralisée q.s.p. | 100 g |

Puis on a appliqué cette composition de teinture sur des mèches de cheveux gris permanentés à 90% de blancs, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration pourpre.

### EXEMPLE 21 :

On a préparé la composition de teinture suivante :

| | |
|---|---|
| Colorant préparé à l'exemple 5 | 0,702g |
| Alcool benzylique | 10,0 g |
| Alcool éthylique | 21,0 g |
| Glycérine | 5,0 g |
| Hydroxyéthylcellulose vendue sous la dénomination Cellosize QP4400H par la société Union Carbide | 2,3 g |
| Acide citrique | 1,4 g |
| Eau déminéralisée q.s.p. | 100 g |

Puis on a appliqué cette composition de teinture sur des mèches de cheveux gris à 90% de blancs et décolorés, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration beige-rosé.

### EXEMPLE 22 :

On a préparé la composition de teinture suivante :

| | |
|---|---|
| Colorant préparé à l'exemple 7 | 0,881g |
| Alcool benzylique | 10,0 g |
| Alcool éthylique | 21,0 g |
| Glycérine | 5,0 g |
| Hydroxyéthylcellulose vendue sous la dénomination Cellosize QP4400H par la société Union Carbide | 2,3 g |
| Acide citrique | 1,4 g |
| Eau déminéralisée q.s.p. | 100 g |

Puis on a appliqué cette composition de teinture sur des mèches de cheveux gris naturels à 90% de blancs, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration cuivrée intense.

### EXEMPLE 23 :

On a préparé la composition de teinture suivante :

| | |
|---|---|
| Colorant préparé à l'exemple 1 | 0,500g |
| Nonylphénol oxyéthyléné par 9 moles d'oxyde d'éthylène | 8,0 g |
| Diéthanolamide d'acide de coprah | 2,0 g |
| Monométhyléther de propylène glycol | 10,0 g |
| 2-amino-2-méthyl-1-propanol q.s.p pH 9 | |
| Eau déminéralisée q.s.p | 100 g |

Puis on a appliqué cette composition de teinture sur des mèches de cheveux gris à 90% de blancs et décolorés, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration saumon clair.

### EXEMPLE 24:

On a préparé la composition de teinture suivante :

| | |
|---|---|
| Colorant préparé à l'exemple 7 | 0,881g |
| Nonylphénol oxyéthyléné par 9 moles d'oxyde d'éthylène | 8,0 g |
| Diéthanolamide d'acide de coprah | 2,0 g |
| Monométhyléther de propylène glycol | 10,0 g |
| 2-amino-2-méthyl-1-propanol q.s.p. pH 9 | |
| Eau déminéralisée q.s.p. | 100 g |

Puis on a appliqué cette composition de teinture sur des mèches de cheveux gris permanentés à 90% de blancs, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration cuivre très clair.

### EXEMPLE 25 :

On a préparé la composition de teinture suivante :

| | |
|---|---|
| Colorant préparé à l'exemple 8 | 1,007g |
| Nonylphénol oxyéthyléné par 9 moles d'oxyde d'éthylène | 8,0 g |
| Diéthanolamide d'acide de coprah | 2,0 g |
| Monométhylèther de propylène glycol | 10,0 g |
| 2-amino-2-méthyl-1-propanol q.s.p. pH 9 | |
| Eau déminéralisée q.s.p. | 100 g |

Puis on a appliqué cette composition de teinture sur des mèches de cheveux gris à 90% de blancs et décolorés, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration blond doré.

### EXEMPLE 26 :

On a préparé la composition de teinture suivante :

| | |
|---|---|
| Colorant préparé à l'exemple 5 | 0,702g |
| Nonylphénol oxyéthyléné par 9 moles d'oxyde d'éthylène | 8,0 g |
| Diéthanolamide d'acide de coprah | 2,0 g |
| Monométhyléther de propylène glycol | 10,0 g |
| 2-amino-2-méthyl-1-propanol q.s.p. pH 9 | |
| Eau déminéralisée q.s.p. | 100 g |

Puis on a appliqué cette composition de teinture sur des mèches de cheveux gris à 90% de blancs et décolorés, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration paille.

### EXEMPLE 27 :

On a préparé la composition de teinture suivante :

| | |
|---|---|
| Colorant préparé à l'exemple 11 | 1,052g |
| Nonylphénol oxyéthyléné par 9 moles d'oxyde d'éthylène | 8,0 g |
| Diéthanolamide d'acide de coprah | 2,0 g |
| Monométhyléther de propylène glycol | 10,0 g |
| 2-amino-2-méthyl-1-propanol q.s.p. pH 9 | |
| Eau déminéralisée q.s.p. | 100 g |

Puis on a appliqué cette composition de teinture sur des mèches de cheveux gris à 90% de blancs et décolorés, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration blond doré.

### EXEMPLE 28 :

On a préparé la composition de teinture suivante :

| | |
|---|---|
| Colorant préparé à l'exemple 12 | 0,934g |
| Alcool benzylique | 10,0 g |
| Alcool éthylique | 21,0 g |
| Glycérine | 5,0 g |
| Hydroxyéthylcellulose vendue sous la dénomination Cellosize QP4400H par la société Union Carbide | 2,3 g |
| Acide citrique | 1,4 g |
| Eau déminéralisée q.s.p. | 100 g |

Puis on a appliqué cette composition de teinture sur des mèches de cheveux gris à 90% de blancs et décolorés, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration rose.

### EXEMPLE 29 :

On a préparé la composition de teinture suivante :

| | |
|---|---|
| Colorant préparé à l'exemple 13 | 0,928g |
| Alcool benzylique | 10,0 g |
| Alcool éthylique | 21,0 g |
| Glycérine | 5,0 g |
| Hydroxyéthylcellulose vendue sous la dénomination Cellosize QP4400H par la société Union Carbide | 2,3 g |
| Acide citrique | 1,4 g |
| Eau déminéralisée q.s.p. | 100 g |

Puis on a appliqué cette composition de teinture sur des mèches de cheveux gris à 90% de blancs et décolorés, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration pourpre clair.

### EXEMPLE 30:

On a préparé la composition de teinture suivante :

| | |
|---|---|
| Colorant préparé à l'exemple 14 | 0,928g |
| Alcool benzylique | 10,0 g |
| Alcool éthylique | 21,0 g |
| Glycérine | 5,0 g |
| QP4400H par la société Union Carbide Hydroxyéthylcellulose vendue sous la dénomination Cellosize | 2,3 g |
| Acide citrique | 1,4 g |
| Eau déminéralisée q.s.p. | 100 g |

Puis on a appliqué cette composition de teinture sur des mèches de cheveux gris permanentés à 90% de blancs, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration beige-rosé intense.

### EXEMPLE 31 :

On a préparé la composition de teinture suivante :

| | |
|---|---|
| Colorant préparé à l'exemple 15 | 0,827g |
| Alcool benzylique | 10,0 g |
| Alcool éthylique | 21,0 g |
| Glycérine | 5,0 g |
| Hydroxyéthylcellulose vendue sous la dénomination Cellosize QP4400H par la société Union Carbide | 2,3 g |
| Acide citrique | 1,4 g |
| Eau déminéralisée q.s.p. | 100 g |

Puis on a appliqué cette composition de teinture sur des mèches de cheveux gris à 90% de blancs et décolorés, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration gris-doré-nacré.

### EXEMPLE 32 :

On a préparé la composition de teinture suivante :

| | |
|---|---|
| Colorant préparé à l'exemple 16 | 1,018g |
| Alcool benzylique | 10,0 g |
| Alcool éthylique | 21,0 g |
| Glycérine | 5,0 g |
| Hydroxyéthylcellulose vendue sous la dénomination Cellosize QP4400H par la société Union Carbide | 2,3 g |
| Acide citrique | 1,4 g |
| Eau déminéralisée q.s.p. | 100 g |

Puis on a appliqué cette composition de teinture sur des mèches de cheveux gris permanentés à 90% de blancs, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration blond-doré.

### EXEMPLE 33 :

On a préparé la composition de teinture suivante :

| | |
|---|---|
| Colorant préparé à l'exemple 17 | 0,976g |
| Alcool benzylique | 10,0 g |
| Alcool éthylique | 21,0 g |
| Glycérine | 5,0 g |
| Hydroxyéthylcellulose vendue sous la dénomination Cellosize QP4400H par la société Union Carbide | 2,3 g |
| Acide citrique | 1,4 g |
| Eau déminéralisée q.s.p. | 100 g |

Puis on a appliqué cette composition de teinture sur des mèches de cheveux gris à 90% de blancs et décolorés, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration cuivre-clair.

### EXEMPLE 34 :

On a préparé la composition de teinture suivante :

| | |
|---|---|
| Colorant préparé à l'exemple 18 | 0,5 g |
| Nonylphénol oxyéthyléné par 9 moles d'oxyde d'éthylène | 8,0 g |
| Diéthanolamide d'acide de coprah | 2,0 g |
| Monométhyléther de propylène glycol | 10,0 g |
| 2-amino-2-méthyl-1-propanol q.s.p. pH 9 | |
| Eau déminéralisée q.s.p. | 100 g |

Puis on a appliqué cette composition de teinture sur des mèches de cheveux gris à 90% de blancs et décolorés, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration beige-rosé.

### EXEMPLE 35 :

On a préparé la composition de teinture suivante :

| | |
|---|---|
| Colorant préparé à l'exemple 19 | 0,838g |
| Alcool benzylique | 10,0 g |
| Alcool éthylique | 21,0 g |
| Glycérine | 5,0 g |
| Hydroxyéthylcellulose vendue sous la dénomination Cellosize QP4400H par la société Union Carbide | 2,3 g |
| Acide citrique | 1,4 g |
| Eau déminéralisée q.s.p. | 100 g |

Puis on a appliqué cette composition de teinture sur des mèches de cheveux gris à 90% de blancs et décolorés, à raison de 3 grammes par gramme de cheveux. On a laissé poser 30 minutes à température ambiante; on a ensuite rincé les mèches à l'eau courante puis on les a séchées.
Les mèches de cheveux ont été teintes dans une coloration bleu lavande argenté.

## Revendications

1. Utilisation à titre de colorants directs dans des, ou pour la préparation de, **compositions cosmétiques destinées à la teinture des cheveux,** de composés de formule (I) ou (II) : 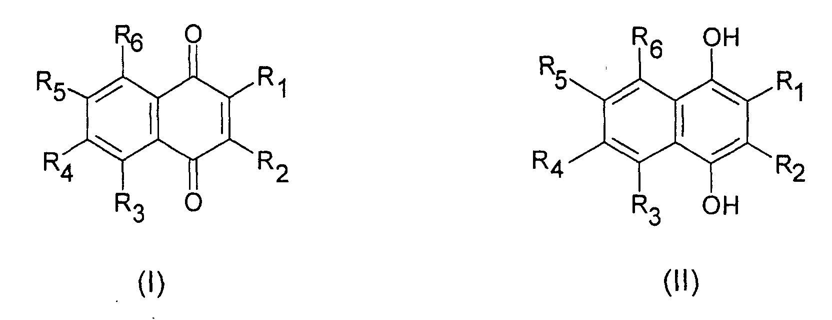 formules (I) ou (II) dans lesquelles :
- R₁ et R₂, identiques ou différents, sont choisis parmi un atome d'hydrogène, un atome d'halogène, un radical OH, un radical alkyle en C₁-C₄, un radical alcoxy en C₁-C₄, ou un radical **Z** de formule (III) définie ci-après,
- R₃ à R₆, identiques ou différents, sont choisis parmi un atome d'hydrogène, un atome d'halogène, un radical OH, un radical alkyle en C₁-C₄, un radical alcoxy en C₁-C₄,
radical **Z** de formule (III) suivante : 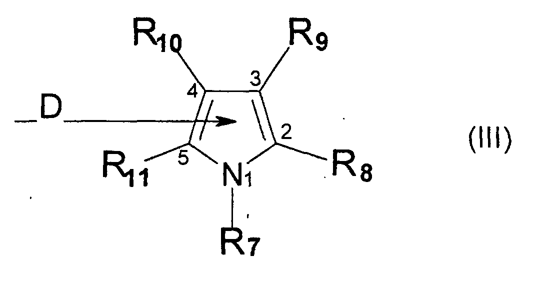 dans laquelle :
- D est une liaison covalente entre le noyau pyrrolique (III) et les noyaux de structure (I) ou (II),
- R₇ à R₁₁, identiques ou différents, désignent l'hydrogène ou un radical alkyle en C₁-C₁₈ linéaire ou ramifié saturé ou insaturé éventuellement substitué par un ou plusieurs atomes d'halogène, par un ou plusieurs radicaux hydroxyles libres ou protégés par une fonction éther ou ester, par un ou plusieurs CN, COOR, NR'R" et leurs sels d'addition d'acide, N(R')COR", -CONR'R"dans lesquels R, R' et R" désignent H ou alkyle(C₁-C₄),
- R₇ à R₁₁, identiques ou différents, désignent également un radical CH₂-C₆H₅ ou SO₂-C₆H₅, un radical COalkyle(C₁-C₄), ou COOH et ses sels , ou COOalkyle(C1-C4), ou CONR'R" avec R' et R" , identiques ou différents, désignent H ou alkyle(C₁-C₄), un radical C₆H₅ qui peut être substitué par un ou plusieurs atomes d'halogène, un ou plusieurs radicaux hydroxy, ou alkyle en C₁-C₄, ou alcoxy en C₁-C₄, et
- R₈ à R₁₁ désignent en outre au moins une liaison D,
étant entendu,
- (i) qu'au moins un des radicaux R₁ ou R₂ désigne un radical Z,
- (ii) que le rattachement de Z aux noyaux de formules (I) ou (II) s'effectue sur l'une quelconque des positions 2 à 5 du noyau pyrrolique,
- (iii) et que les radicaux R₈ et R₁₀ ou les radicaux R₉ et R₁₁ ne désignent pas simultanément un composé de formule (I).

2. Utilisation selon la revendication 1, **caractérisée par le fait que** dans la formule (I) :
- R₁ est un radical Z de formule (III) pour laquelle R₇ est hydrogène, méthyle, n-pentyle, isopentényle, n-octyle, n-hexadécyle, CH₂-C₆H₅, (CH₂)₂-CN, (CH₂)₂-OH, (CH₂)₂-NH-COCH₃, C(CH₃)(CH₂OH)₂, R₈ est hydrogène, CH₃, COOC₂H₅, CH₂-COOH, CH₂-COOCH₃, 2-(1,4-naphtoquinonyle), 2-(8-hydroxy-1,4-naphtoquinonyle), R₉ est hydrogène, CH₃, C₂H₅, COCH₃, COOH ou COOCH₃, R₁₀ est hydrogène, CH₃, 2-(1,4-naphtoquinonyle), ou 2-(8-hydroxy-1,4-naphtoquinonyle), R₁₁ est CH₃ ou 2-(1,4-naphtoquinonyle) ou 2-(8-hydroxy-1,4-naphtoquinonyle),
- R₂ est hydrogène,
- R₃ à R₅ sont hydrogène,
- R₆ est hydrogène ou un radical OH.

3. Utilisation selon la revendication **2**, **caractérisée par le fait que** les composés de formule (I) sont les suivants :
2-(1-H-pyrrol-2-yl)-[1,4]-naphtoquinone,
2-(1-méthyl-1H-pyrrol-2-yl)-[1,4]-naphtoquinone,
2-(1-pentyl-1H-pyrrol-2-yl)-[1,4]-naphtoquinone,
2-(1-octyl-1H-pyrrol-2-yl)-[1,4]-naphtoquinone,
2-(1-hexadécyl-1H-pyrrol-2-yl)-[1,4]-naphtoquinone,
2-(1-benzyl-1H-pyrrol-2-yl)-[1,4]-naphtoquinone,
2-(4-éthyl-3,5-diméthyl-1H-pyrrol-2-yl)-[1,4]-naphtoquinone,
3-[2-(1,4-dioxo-1,4-dihydro-naphtalen-2-yl)-pyrrol-1-yl]-propionitrile,
1-méthyl-2,5-bis-(1,4-naphtoquinone-2-yl)-pyrrole,
1-pentyl-2,5-bis-(1,4-naphtoquinone-2-yl)-pyrrole,
1-octyl-2,5-bis-(1,4-naphtoquinone-2-yl)-pyrrole,
1-hexadécyl-2,5-bis-(1,4-naphtoquinone-2-yl)-pyrrole,
1-benzyl-2,5-bis-(1,4-naphtoquinone-2-yl)-pyrrole,
ester méthylique de l'acide [5-(1,4-dioxo-1,4-dihydro-naphtalen-2-yl)-1-méthyl-1-H-pyrrol-2-yl]-acétique,
ester méthylique de l'acide 4-(1,4-dioxo-1,4-dihydro-naphtalen-2-yl)-2,5-diméthyl-1-H-pyrrole-3-carboxylique,
ester méthylique de l'acide 4-(1,4-dioxo-1,4-dihydro-naphtalen-2-yl)-1,2,5-triméthyl-1-H-pyrrole-3-carboxylique,
2-(2,5-diméthyl-1-H-pyrrol-3-yl)-8-hydroxy-[1,4]-naphtoquinone,
ester méthylique de l'acide 4-(8-hydroxy-1,4-dioxo-1,4-dihydro-naphtalen-2-yl)-1,2,5-triméthyl-1-H-pyrrole-3-carboxylique,
ester méthylique de l'acide 4-(8-hydroxy-1,4-dioxo-1,4-dihydro-naphtalen-2-yl)-2,5-diméthyl-1-H-pyrrole-3-carboxylique,
N-{2-[2-(1,4-dioxo-1,4-dihydro-naphtalen-2-yl)-pyrrol-1-yl]-éthyl}-acétamide,
2-{N-[(2-hydroxy-1-hydroxyméthyl-1-méthyl)-éthyl]-1-H-pyrrol-2-yl1}-[1,4]-naphtoquinone,
2-[N-(β-hydroxyéthyl)-1 -H-pyrrol-2-yl]-[1,4]-naphtoquinone,
acide 2-carboxyméthyl-5-(1,4-dioxo-1,4-dihydro-naphtalen-2-yl)-1,4-diméthyl-1-H-pyrrole-3-carboxylique,
acide 4-(1,4-dioxo-1,4-dihydro-naphtalen-2-yl)-2,5-diméthyl-1-H-pyrrole-3-carboxylique,
ester éthylique de l'acide 4-(1,4-dioxo-1,4-dihydro-naphtalen-2-yl)-3,5-diméthyl-1-H-pyrrole-2-carboxylique,
2-(4-acétyl-3,5-diméthyl-1-H-pyrrol-2-yl)- [1,4]-naphtoquinone.

4. Utilisation selon l'une quelconque des revendications 1 à 2 3, **caractérisée par le fait que** le composé de formule (II) est choisi parmi le 2-(1-méthyl-1-H-pyrrol-2-yl)-[1,4]-dihydroxy-naphtalène et l'ester méthylique de l'acide [5-(1,4-dihydroxy-naphtalen-2-yl)-1-méthyl-1-H-pyrrol-2-yl]-acétique.

5. Composés de formule (I) telle que définie dans la revendication 1, à l'exception des composés suivants :
2-(1-benzènesulfonyl-1H-pyrrol-2-yl)-[1,4]-naphtoquinone,
2-(1-méthyl-1H-pyrrol-2-yl)-[1,4]-naphtoquinone,
2-(1-benzyl-1-H-pyrrol-2-yl)-3-méthoxy-[1,4]-naphtoquinone,
2-(1-éthyl-1-H-pyrrol-2-yl)-3-méthoxy-[1,4]-naphtoquinone,
2-(1-H-pyrrol-2-yl)-3-méthoxy-[1,4]-naphtoquinone,
2-(1-méthyl-1-H-pyrrol-2-yl)-3-bromo-[1,4]-naphtoquinone,
2-(1-méthyl-1-H-pyrrol-2-yl)-3-méthyl-[1,4]-naphtoquinone,
2-(1-méthyl-1-H-pyrrol-2-yl)-3-méthoxy-[1,4]-naphtoquinone,
2-(1,2,5-triméthyl-1-H-pyrrol-4-yl)-3-chloro-[1,4]-naphtoquinone,
2-(2,5-diéthyl-1-méthyl-1-H-pyrrol-3-yl)-[1,4]-naphtoquinone,
1-méthyl-2,5-bis-(1,4-naphtoquinone-2-yl)-pyrrole,
ainsi que des composés de formule (IV) suivante : 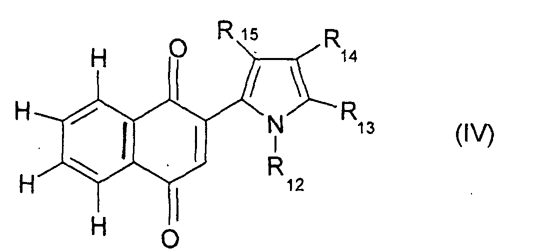 formule dans laquelle R₁₂ désigne H, alkyle, aryle ou aralkyle et R₁₃, R₁₄ et R₁₅, identiques ou différents sont H, alkyle, phényle, sulfonyle substitué par un phényle.

6. Composés de formule (II) telle que définie dans la revendication 1, à l'exception du 2-(1-méthyl-1-H-pyrrol-2-yl)-[1,4]-dihydroxy-naphtalène.

7. Composition cosmétique destinée à la teinture des cheveux, **caractérisée par le fait qu'**elle comprend, dans un milieu cosmétiquement acceptable, 0,01 à 10% en poids par rapport au poids total de la composition d'au moins un des composés de formule (I) ou (II) définis à l'une quelconque des revendications 1-**6**.

8. Composition selon la revendication **7, caractérisée par le fait qu'**elle a un pH compris entre 2 et 11.

9. Composition selon les revendications **7 à 8** , **caractérisée par le fait que** le milieu cosmétiquement acceptable est constitué par de l'eau et/ou des solvants organiques tels que des alcools, des glycols, des éthers de glycols, dans des proportions comprises entre 0,5 et 25% en poids par rapport au poids total de la composition.

10. Procédé de teinture des cheveux, par coloration directe, **caractérisé par le fait qu'**on applique une composition tinctoriale renfermant au moins un composé de formule (I) ou (II) tel que défini à l'une quelconque des revendications 1 à **6,** sur les fibres kératiniques, sèches ou humides, et qu'après avoir éventuellement laissé agir la composition sur les fibres pendant un temps de pose allant de 3 à 60 minutes, on sèche ces fibres, après un rinçage éventuel.

## Patentansprüche

1. Verwendung von Verbindungen der Formeln I oder II als Direktfarbstoffe in kosmetischen Zusammensetzungen zum Färben der Haare oder zur Herstellung dieser Zusammensetzungen: 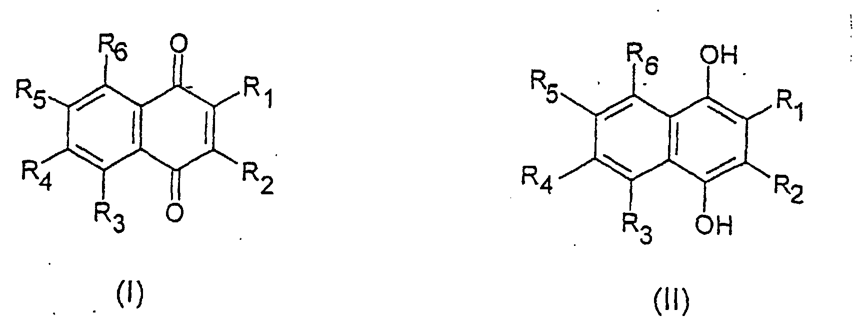 wobei in den Formeln I oder II bedeuten:
- die Gruppen R₁ und R₂, die identisch oder voneinander verschieden sind, ein Wasserstoffatom, ein Halogenatom, die OH-Gruppe, eine C₁₋₄-Alkylgruppe, eine C₁₋₄-Alkoxygruppe oder eine Gruppe Z der nachstehend definierten Formel III,
- die Gruppen R₃ bis R₆, die identisch oder voneinander verschieden sind, ein Wasserstoffatom, ein Halogenatom, die OH-Gruppe, eine C₁₋₄-Alkylgruppe, eine C₁₋₄-Alkoxygruppe oder eine Gruppe Z der folgenden Formel III 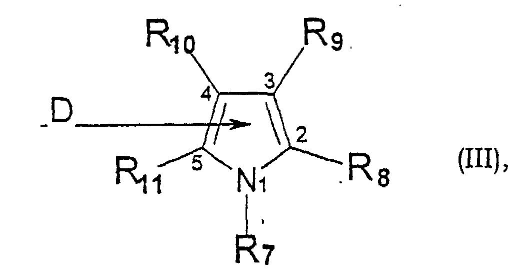
worin bedeuten:
- D eine kovalente Bindung zwischen dem Pyrrolring (III) und den Ringen der Struktur (I) oder (II),
- die Gruppen R₇ bis R₁₁, die identisch oder voneinander verschieden sind, Wasserstoff oder eine geradkettige oder verzweigte C₁₋₁₈-Alkylgruppe, gesättigt oder ungesättigt die ggf. mit einem oder mehreren Halogenatomen, mit einer oder mehreren freien oder in Form von Ether- oder Estergruppen geschützten Hydroxygruppen oder mit einer oder mehreren der folgenden Gruppen substituiert ist: -CN, -COOR, -NR'R" und deren Additionssalzen mit einer Säure, -N(R')COR" und -CONR'R", wobei die Gruppen R, R' und R" H oder C₁₋₄-Alkyl bedeuten,
die Gruppen R₇ bis R₁₁, die identisch oder voneinander verschieden sind, können auch bedeuten: CH₂-C₆H₅, SO₂-C₆H₅, eine Gruppe CO-C₁₋₄-alkyl, die Gruppe COOH und ihre Salze, COO-C₁₋₄-alkyl, CONR'R", wobei die Gruppen R' und R", die identisch oder voneinander verschieden sind, H oder C₁₋₄-Alkyl bedeuten, eine Gruppe C₆H₅, die mit einem oder mehreren Halogenatomen oder einer oder mehreren Hydroxygruppen, C₁₋₄-Alkyl oder C₁₋₄-Alkoxy substituiert sein kann, und
- die Gruppen R₈ bis R₁₁ mindestens eine Bindung D, mit der Maßgabe, dass
- (i) mindestens eine der Gruppen R₁ oder R₂ Z bedeutet,
- (ii) die Bindung der Gruppe Z an die Ringe der Formel (I) oder (II) an einer beliebigen Stellung 2 bis 5 des Pyrrolrings erfolgt, und
- (üi) die Gruppen R₈ und R₁₀ oder die Gruppen R₉ und R₁₁ nicht gleichzeitig eine Verbindung der Formel (I) bedeuten.

2. Verwendung nach Anspruch 1, **dadurch gekennzeichnet, daß** in der Formel I bedeuten:
- R₁ eine Gruppe Z der Formel (III), wobei R₇ Wasserstoff, Methyl, n-Pentyl, Isopentenyl, n-Octyl, n-Hexadecyl, CH₂-C₆H₅, (CH₂)₂-CN, (CH₂)₂-OH, (CH₂)₂-NH-COCH₃ und C(CH₃)(CH₂OH)₂ bedeutet, R₈ Wasserstoff, CH₃, COOC₂H₅, CH₂-COOH, CH₂-COOCH₃, 2-(1,4-Naphthochinonyl) und 2-(8-Hydroxy-1,4-naphthochinonyl) bedeutet, R₉ Wasserstoff, CH₃, C₂H₅, COCH₃, COOH oder COOCH₃ ist, R₁₀ Wasserstoff, CH₃, 2-(1,4-Naphthochinonyl) oder 2-(8-Hydroxy-1,4-naphthochinonyl) bedeutet und R₁₁ CH₃ oder 2-(1,4- Naphthochinonyl) oder 2-(8-Hydroxy-1,4-naphthochinonyl) ist,
- R₂ Wasserstoff,
- R₃ bis R₅ Wasserstoff, und
- R₆ Wasserstoff oder OH.

3. Verwendung nach Anspruch 2, **dadurch gekennzeichnet, daß** es sich bei den Verbindungen der Formel I um die folgenden Verbindungen handelt:
- 2-(1-H-Pyrrol-2-yl)-[1,4]-naphthochinon,
- 2-(1-Methyl-1H-pyrrol-2-yl)-[1,4]-naphthochinon,
- 2-(1-Pentyl-1H-pyrrol-2-yl)-[1,4]-naphthochinon,
- 2-(1-Octyl-1H-pyrrol-2-yl)-[1,4]-naphthochinon,
- 2-(1-Hexadecyl-1H-pyrrol-2-yl)-[1,4]-naphthochinon,
- 2-(1-Benzyl-1H-pyrrol-2-yl)-[1,4]-naphthochinon,
- 2-(4-Ethyl-3,5-dimethyl-1H-pyrrol-2-yl)-[1,4]-naphthochinon,
- 3-[2-(1,4-Dioxo-1,4-dihydronaphthalin-2-yl)-pyrrol-1-yl]-propionitril,
- 1-Methyl-2,5-bis(1,4-naphthochinon-2-yl)-pyrrol,
- 1-Pentyl-2,5-bis(1,4-naphthochinon-2-yl)-pyrrol,
- 1-Octyl-2,5-bis(1,4-naphthochinon-2-yl)-pyrrol,
- 1-Hexadecyl-2,5-bis(1,4-naphthochinon-2-yl)-pyrrol,
- 1-Benzyl-2,5-bis(1,4-naphthochinon-2-yl)-pyrrol,
- [5-(1,4-Dioxo-1,4-dihydronaphthalin-2-yl)-1-methyl-1-H-pyrrol-2-yl]-essigsäuremethylester,
- 4-(1,4-Dioxo-1,4-dihydronaphthalin-2-yl)-2,5-dimethyl-1-Hpyrrol-3-carbonsäuremethylester,
- 4-(1,4-Dioxo-1,4-dihydronaphthalin-2-yl)-1,2,5-trimethyl-1-Hpyrrol-3-carbonsäuremethylester,
- 2-(2,5-Dimethyl-1-H-pyrrol-3-yl)-8-hydroxy-[1,4]-naphthochinon,
- 4-(8-Hydroxy-1,4-dioxo-1,4-dihydronaphthalin-2-yl)-1,2,5-trimethyl-1-H-pyrrol-3-carbonsäuremethylester,
- 4-(8-Hydroxy-1,4-dioxo-1,4-dihydronaphthalin-2-yl)-2, 5-dimethyl-1-H-pyrrol-3-carbonsäuremethylester,
- N-{2-[2-( 1,4-Dioxo-1,4-dihydronaphthalin-2-yl)-pyrrol-1-yl]-ethyl}-acetamid,
- 2-{N- [(2-Hydroxy-1-hydroxymethyl-1-methyl)-ethyl]-1-H-pyrrol-2-yl}-[1,4]-naphthochinon,
- 2-[N-(β-Hydroxyethyl)-1-H-pyrrol-2-yl]-[1,4]-naphthochinon,
- 2-Carboxymethyl-5-(1,4-dioxo-1,4-dihydronaphthalin-2-yl)-1,4-dimethyl-1-H-pyrrol-3-carbonsäure,
- 4-(1,4-Dioxo-1,4-dihydronaphthalin-2-yl)-2,5-dimethyl-1-Hpyrrol-3-carbonsäure,
- 4-(1,4-Dioxo-1,4-dihydronaphthalin-2-yl)-3,5-dimethyl-1-Hpyrrol-2-carbonsäureethylester und
- 2-(4-Acetyl-3,5-dimethyl-1-H-pyrrol-2-yl)-[1,4]-naphthochinon.

4. Verwendung nach einem der Ansprüche 1 bis 2, **dadurch gekennzeichnet, daß** die Verbindung der Formel II unter 2-(1-Methyl-1-H-pyrrol-2-yl)-[1,4]-dihydroxynaphthalin und dem Methylester von [5-(1,4-Dihydroxynaphthalin-2-yl)-1-methyl-1-Hpyrrol-2-yl]-essigsäure ausgewählt ist.

5. Verbindungen der Formel I nach Anspruch 1, mit Ausnahme der folgenden Verbindungen:
2-(1-Methyl-1H-pyrrol-2-yl)-[1,4]-naphthochinon,
2-(1-Benzyl-1-H-pyrrol-2-yl)-3-methoxy-[1,4]-naphthochinon,
2-(1-Ethyl-1-H-pyrrol-2-yl)-3-methoxy-[1,4]-naphthochinon,
2-(1-H-Pyrrol-2-yl)-3-methoxy-[1,4]-naphthochinon,
2-(1-Methyl-1-H-pyrrol-2-yl)-3-brom-[1,4]-naphthochinon,
2-(1-Methyl-1-H-pyrrol-2-yl)-3-methyl-[1,4]-naphthochinon,
2-(1-Methyl-1-H-pyrrol-2-yl)-3-methoxy-[1,4]-naphthochinon,
2-(1,2,5-Trimethyl-1-H-pyrrol-4-yl)-3-chlor-[1,4]-naphthochinon,
2-(2,5-Diethyl-1-methyl-1-H-pyrrol-3-yl)-[1,4]-naphthochinon,
1-Methyl-2,5-bis(1,4-naphthochinon-2-yl)-pyrrol,
sowie Verbindungen der folgenden Formel (IV): 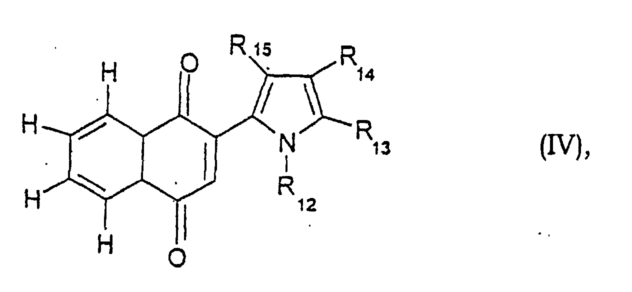 wobei in der Formel R₁₂ H, Alkyl, Aryl oder Aralkyl bedeutet und die Gruppen R₁₃, R₁₄ und R₁₅, die identisch oder voneinander verschieden sind, H, Alkyl, Phenyl oder mit Phenyl substituiertes Sulfonyl bedeuten.

6. Verbindungen der Formel II nach Anspruch 1, wobei das 2-(1-Methyl-1-H-pyrrol-2-yl)-[1,4]-dihydroxynaphthalin ausgenommen ist.

7. Kosmetische Zusammensetzung zum Färben der Haare, **dadurch gekennzeichnet, daß** sie in einem kosmetisch akzeptablen Medium 0,01 bis 10 Gew.-% mindestens einer Verbindung der Formel I oder II nach einem der Ansprüche 1 bis 6, bezogen auf das Gesamtgewicht der Zusammensetzung, enthält.

8. Zusammensetzung nach Anspruch 7, **dadurch gekennzeichnet, daß** sie einen pH-Wert im Bereich von 2 bis 11 aufweist.

9. Zusammensetzung nach den Ansprüchen 7 bis 8, **dadurch gekennzeichnet, daß** das kosmetisch akzeptable Medium aus Wasser und/oder organischen Lösungsmitteln, wie Alkoholen, Glykolen oder Glykolethern, in Mengenanteilen von 0,5 bis 25 Gew.-%, bezogen auf das Gesamtgewicht der Zusammensetzung, besteht.

10. Verfahren zum Färben von Haaren durch direkte Färbung, **dadurch gekennzeichnet, daß** eine Farbmittelzusammensetzung, die mindestens eine Verbindung der Formel I oder II nach einem der Ansprüche 1 bis 6 enthält, auf die trockenen oder feuchten Keratinfasern aufgetragen wird, die Zusammensetzung ggf. während einer Einwirkzeit von 3 bis 60 min auf die Fasern einwirken gelassen wird und die Fasern nach einem fakultativen Spülschritt getrocknet werden.

## Claims

1. Use as direct dyes in or for the preparation of cosmetic compositions intended for dyeing hair of compounds of formula (I) or (II): 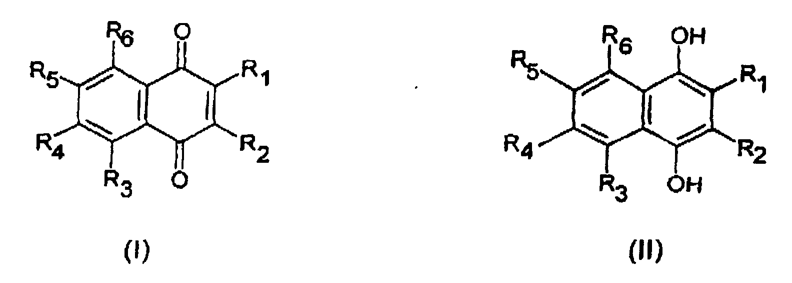 in which formulae (I) or (II):
- R₁ and R₂, which are identical or different, are chosen from a hydrogen atom, a halogen atom, an OH radical, a C₁-C₄ alkyl radical, a C₁-C₄ alkoxy radical and a radical Z of formula (III) defined below,
- R₃ to R₆, which are identical or different, are chosen from a hydrogen atom, a halogen atom, an OH radical, a C₁-C₄ alkyl radical and a C₁-C₄ alkoxy radical,
radical Z of formula (III) below: 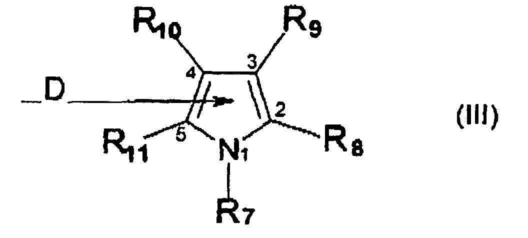 in which:
- D is a covalent bond between the pyrrole ring (III) and the rings of structure (I) or (II),
- R₇ to R₁₁, which are identical or different, denote hydrogen or a linear or branched saturated or unsaturated C₁-C₁₈ alkyl radical optionally substituted with one or more halogen atoms, with one or more hydroxyl radicals which are free or protected by an ether or ester function, or with one or more of CN, COOR, NR'R" and their acid addition salts, N(R')COR", -CONR'R" in which R, R' and R" denote H or (C₁-C₄) alkyl,
- R₇ to R₁₁, which are identical or different, also denote a CH₂-C₆H₅ or SO₂-C₆H₅ radical, a radical CO(C₁-C₄)alkyl, or COOH and its salts, or COO(C₁-C₄)alkyl, or CONR'R" with R' and R", which are identical or different, denoting H or (C₁-C₄) alkyl, a C₆H₅ radical which may be substituted with one or more halogen atoms or one or more hydroxyl, C₁-C₄ alkyl or C₁-C₄ alkoxy radicals, and
R₈ to R₁₁ also denote at least one bond D,
it being understood
- (i) that at least one of the radicals R₁ or R₂ denotes a radical Z,
- (ii) that the attachment of Z to the rings of formulae (I) or (II) is carried out on any of the positions 2 to 5 of the pyrrole ring,
- (iii) and that the radicals R₈ and R₁₀ or the radicals R₉ and R₁₁ do not simultaneously denote a compound of formula (I).

2. use according to Claim 1, **characterized in that**, in formula (I):
- R₁ is a radical z of formula (III) for which R₇ is hydrogen, methyl, n-pentyl, isopentenyl, n-octyl, n-hexadecyl, CH₂-C₆H₅, (CH₂)₂-CN, (CH₂)₂-OH, (CH₂)₂-NH-COCH₃, C(CH₃)(CH₂OH)₂, R₈ is hydrogen, CH₃, COOC₂H₅, CH₂-COOH, CH₂-COOCH₃, 2-(1,4-naphthoquinonyl), 2-(8-hydroxy-1,4-naphthoquinonyl), R₉ is hydrogen, CH₃, C₂H₅, COCH₃, COOH or COOCH₃, R₁₀ is hydrogen, CH₃, 2-(1,4-naphthoquinonyl) or 2-(8-hydroxy-1,4-naphthoquinonyl), R₁₁ is CH₃, 2-(1,4-naphthoquinonyl) or 2-(8-hydroxy-1,4-naphthoquinonyl),
- R₂ is hydrogen,
- R₃ to R₅ are hydrogen,
- R₆ is hydrogen or an OH radical.

3. Use according to Claim 2, **characterized in that** the compounds of formula (I) are the following:
- 2-(1H-pyrrol-2-yl)-[1,4]-naphthoquinone,
- 2-(1-methyl-1H-pyrrol-2-yl)-[1,4]-naphthoquinone,
- 2-(1-pentyl-lH-pyrrol-2-yl)-(1,4]-naphthoquinone,
- 2-(1-octyl-1H-pyrrol-2-yl)-[1,4]-naphthoquinone,
- 2-(1-hexadecyl-1H-pyrrol-2-yl)-[1,4]-naphthoquinone,
- 2-(1-benzyl-lH-pyrrol-2-yl)-[1,4]-naphthoquinone,
- 2-(4-ethyl-3,5-dimethyl-1H-pyrrol-2-yl)-[1,4]-naphthoquinone,
- 3-[2-(1,4-dioxo-1,4-dihydronaphthalen-2-yl)pyrrol-1-yl]propionitrile,
- 1-methyl-2,5-bis(1,4-naphthoquinon-2-yl)pyrrole,
- 1-pentyl-2,5-bis(1,4-naphthoquinon-2-yl)pyrrole,
- 1-octyl-2,5-bis(1,4-naphthoquinon-2-yl)pyrrole,
- 1-hexadecyl-2,5-bis(1,4-naphthoquinon-2-yl)pyrrole,
- 1-benzyl-2,5-bis(1,4-naphthoquinon-2-yl)pyrrole,
- methyl [5-(1,4-dioxo-1,4-dihydronaphthalen-2-yl)-1-methyl-1H-pyrrol-2-yl]acetate,
- methyl 4-(1,4-dioxo-1,4-dihydronaphthalen-2-yl)-2,5-dimethyl-1H-pyrrole-3-carboxylate.
- methyl 4-(1,4-dioxo-1,4-dihydronaphthalen-2-yl)-1,2,5-trimethyl-1H-pyrrole-3-carboxylate,
- 2-(2,5-dimethyl-1H-pyrrol-3-yl)-8-hydroxy-[1,4]-naphthoquinone,
- methyl 4-(8-hydroxy-1,4-dioxo-1,4-dihydronaphthalen-2-yl)-1,2,5-trimethyl-1H-pyrrole-3-carboxylate,
- methyl 4-(8-hydroxy-1,4-dioxo-1,4-dihydronaphthalen-2-yl)-2,5-dimethyl-lH-pyrrole-3-carboxylate,
- N-{2-[2-(1,4-dioxo-1,4-dihydronaphthalen-2-yl)pyrrol-1-yl]ethyl}acetamide,
- 2-{N-[(2-hydroxy-1-hydroxymethyl-1-methyl)ethyl]-1H-pyrrol-2-yl}-[1,4]-naphthoquinone,
- 2-[N-(β-hydroxyethyl)-1H-pyrrol-2-yl]-[1,4]-naphthoquinone,
- 2-carboxymethyl-5-(1, 4-dioxo-1,4-dihydronaphthalen-2-yl)-1,4-dimethyl-1H-pyrrole-3-carboxylic acid,
- 4-(1,4-dioxo-1,4-dihydronaphthalen-2-yl)-2,5-dimethyl-1H-pyrrole-3-carboxylic acid,
- ethyl 4-(1,4-dioxo-1,4-dihydronaphthalen-2-yl)-3,5-dimethyl-1H-pyrrole-2-carboxylate,
- 2-(4-acetyl-3,5-dimethyl-1H-pyrrol-2-yl)-[1,4]-naphthoquinone.

4. Use according to any one of Claims 1 to 2, **characterized in that** the compound of formula (II) is chosen from 2-(1-methyl-1H-pyrrol-2-yl)-[1,4]-dihydroxynaphthalene and methyl [5-(1,4-dihydroxynaphthalen-2-yl)-1-methyl-1H-pyrrol-2-yl]acetate.

5. Compounds of formula (I) as defined in Claim 1, except for the following compounds:
2-(1-methyl-1H-pyrrol-2-yl)-[1,4]-naphthoquinone,
2-(1-benzyl-1H-pyrrol-2-yl)-3-methoxy-[1,4]-naphthoquinone,
2-(1-ethyl-1H-pyrrol-2-yl)-3-methoxy-[1,4]-naphthoquinone,
2-(1H-pyrrol-2-yl)-3-methoxy-[1,4]-naphthoquinone,
2-(1-methyl-1H-pyrrol-2-yl)-3-bromo-[1,4]-naphthoquinone,
2-(1-methyl-1H-pyrrol-2-yl)-3-methyl-[1,4]-naphthoquinone,
2-(1-methyl-1H-pyrrol-2-yl)-3-methoxy-[1,4]-naphthoquinone,
2-(1,2,5-trimethyl-1H-pyrrol-4-yl)-3-chloro-[1,4]-naphthoquinone,
2-(2,5-diethyl-1-methyl-1H-pyrrol-3-yl)-[1,4]-naphthoquinone,
1-methyl-2,5-bis(1,4-naphthoquinon-2-yl)pyrrole,
and also compounds of formula (IV) below: 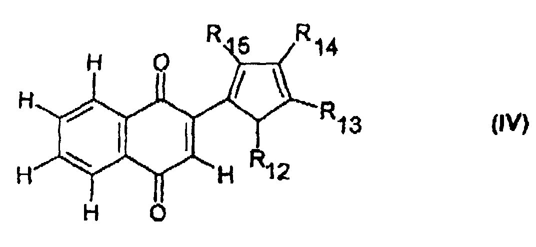 in which formula R₁₂ denotes H, alkyl, aryl or aralkyl and R₁₃, R₁₄ and R₁₅, which are identical or different, are H, alkyl, phenyl, sulphonyl substituted with a phenyl.

6. Compounds of formula (II) as defined in Claim 1. except for 2-(1-methyl-1H-pyrrol-2-yl)-[1,4]-dihydroxynaphthalene.

7. Cosmetic composition intended for dyeing hair, **characterized in that** it comprises, in a cosmetically acceptable medium, 0.01 to 10% by weight, relative to the total weight of the composition, of at least one of the compounds of formula (I) or (II) defined in any one of Claims 1-6.

8. Composition according to Claim 7, **characterized in that** it has a pH of between 2 and 11.

9. Composition according to Claims 7 to 8, **characterized in that** the cosmetically acceptable medium consists of water and/or organic solvents such as alcohols, glycols or glycol ethers, in proportions of between 0.5 and 25% by weight relative to the total weight of the composition.

10. Process for dyeing hair by direct dyeing, **characterized in that** a dye composition containing at least one compound of formula (I) or (II) as defined in any one of Claims 1 to 6 is applied to wet or dry keratin fibres, and **in that**, after the composition has optionally been left to act on the fibres for an exposure time ranging from 3 to 60 minutes, these fibres are dried, after optional rinsing.
